# EUROPEAN PATENT SPECIFICATION

(11) **EP 4 200 871 B1**
(45) Date of publication and mention of the grant of the patent: **10.06.2026**
(21) Application number: 21766153.7
(22) Date of filing: 20.08.2021
(51) Int. Cl.: G16H 20/13, G16H 40/63, G16H 50/30, A61M 15/00, A61M 16/00, A61B 5/087

(54) **INHALER SYSTEMS AND CORRESPONDING METHODS**
INHALATORSYSTEME UND ENTSPRECHENDE VERFAHREN
SYSTÈMES D'INHALATEUR ET MÉTHODES CORRESPONDANTES

(30) Priority: 21.08.2020 GB 202013129
(43) Date of publication of application: 28.06.2023
(73) Proprietor: Norton (Waterford) Limited, Waterford, X91 WK68 (IE)
(72) Inventor: MILTON-EDWARDS, Mark, Macclesfield Cheshire SK10 26B (GB); SAFIOTI, Guilherme, 611 72 Stigtomta (SE); REICH, Michael, 4650442 Herzliya (IL)
(74) Representative: Elkington and Fife LLP
(86) International application number: PCT/EP2021/073171
(87) International publication number: WO 2022/038275

(56) References cited:
- US-A1- 2010 250 280
- US-A1- 2014 206 949
- US-A1- 2019 102 522
- US-A1- 2019 385 727

## Description

### FIELD OF THE INVENTION

This disclosure relates to an inhaler system, and particularly systems and methods for warning of an impending respiratory disease exacerbation.

### BACKGROUND OF THE INVENTION

Many respiratory diseases, such as asthma or chronic obstructive pulmonary disease (COPD), are lifelong conditions where treatment involves the long-term administration of medicaments to manage the patients' symptoms and to decrease the risks of irreversible changes. There is currently no cure for diseases like asthma and COPD. Treatment takes two forms. First, a maintenance aspect of the treatment is intended to reduce airway inflammation and, consequently, control symptoms in the future. The maintenance therapy is typically provided by inhaled corticosteroids, alone or in combination with long-acting bronchodilators and/or muscarinic antagonists. Secondly, there is also a rescue (or reliever) aspect of the therapy, where patients are given rapid-acting bronchodilators to relieve acute episodes of wheezing, coughing, chest tightness and shortness of breath. Patients suffering from a respiratory disease, such as asthma or COPD may also experience episodic flare-ups, or exacerbations, in their respiratory disease, where symptoms rapidly worsen. In the worst case, exacerbations may be life-threatening.

The published patent application US 2019/385727 A1 discloses a respiratory system and method that monitors medication flow.

The ability to identify an impending respiratory disease exacerbation would improve action plans and provide opportunities for pre-emptive treatment, before the patient's condition requires, for example, unscheduled visits to or from a medical practitioner, hospital admission and administering of systemic steroids.

There is therefore a need in the art for improved methods for warning of an impending respiratory disease exacerbation.

### SUMMARY OF THE INVENTION

Accordingly, the present disclosure provides a system for providing an output for warning of a respiratory disease exacerbation in a subject.

An exemplary system comprises a first inhaler for delivering a rescue medicament to the subject. The first inhaler has a use determination system configured to determine a rescue inhalation performed by the subject using the first inhaler.

The exemplary system may further comprise an optional second inhaler for delivering a maintenance medicament to the subject during a routine inhalation.

A sensor system is configured to measure a parameter relating to airflow during the rescue inhalation and/or during the routine inhalation using the second inhaler when included in the system.

The exemplary system further comprises a processing module configured to monitor the frequency of the determined rescue inhalations. The processing module also monitors the parameter as a function of time.

The processing module is further configured to provide the output for warning of the respiratory disease exacerbation if the parameter as a function of time is indicative of the lung condition of the subject deteriorating over a first time period, and the frequency of rescue inhalations is higher during a second time period than during the first time period, the second time period being subsequent to the first time period.

Use of both the number of rescue inhalations and the parameter relating to airflow during the rescue and/or routine inhalations leads to a more accurate warning system for predicting the respiratory disease exacerbation than, for example, a system which neglects either one of these factors.

Moreover, it has been found from analysis of subjects' inhaler use over time that lung condition deterioration as indicated by change in the parameter tends to be followed in time by increased frequency of rescue medicament usage prior to an exacerbation. This observation is utilized in the present system such that warning of an impending respiratory disease exacerbation may be made more reliable.

It is noted that the deterioration in the lung condition of the subject, as indicated by the change in the parameter as a function of time, may also continue during the second time period.

### BRIEF DESCRIPTION OF THE DRAWINGS

The present invention will now be described in more detail with reference to the accompanying drawings, which are not intended to be limiting:
Fig. 1 shows a block diagram of a system according to an example;
Fig. 2 shows a system according to another example;
Fig. 3 shows a graph of flow rate versus time during use of an inhaler;
Fig. 4 shows front and rear views of the exterior of an inhaler according to an example;
Fig. 5 shows an uppermost surface of the top cap of the inhaler shown in Fig. 4;
Fig. 6 schematically depicts pairing the inhaler shown in Fig. 4 with a user device;
Fig. 7 provides a flowchart of a method according to an example;
Fig. 8 shows a timeline of inhalations of a rescue medicament;
Fig. 9 shows graphs of peak inhalation flow, inhalation volume, and number of rescue inhalations versus time for a subject suffering from COPD;
Fig. 10 shows graphs of peak inhalation flow, inhalation volume, and number of rescue inhalations versus time for a subject suffering from asthma;
Fig. 11 shows graphs of inhalation volume, and number of rescue inhalations versus time for a subject suffering from asthma;
Fig. 12 shows graphs of inhalation volume, and number of rescue inhalations versus time for a subject suffering from COPD;
Fig. 13 shows a front perspective view of an inhaler;
Fig. 14 shows a cross-sectional interior perspective view of the inhaler shown in Fig. 13;
Fig. 15 provides an exploded perspective view of the example inhaler shown in Fig. 13;
Fig. 16 provides an exploded perspective view of a top cap and electronics module of the inhaler shown in Fig. 13; and
Fig. 17 shows a graph of airflow rate through the example inhaler shown in Fig. 13 versus pressure.

### DETAILED DESCRIPTION OF THE INVENTION

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the figures to indicate the same or similar parts.

Asthma and COPD are chronic inflammatory disease of the airways. They are both characterized by variable and recurring symptoms of airflow obstruction and bronchospasm. The symptoms include episodes of wheezing, coughing, chest tightness and shortness of breath.

The symptoms are managed by avoiding triggers and by the use of medicaments, particularly inhaled medicaments. The medicaments include inhaled corticosteroids (ICSs) and bronchodilators.

Inhaled corticosteroids (ICSs) are steroid hormones used in the long-term control of respiratory disorders. They function by reducing the airway inflammation. Examples include budesonide, beclomethasone (dipropionate), fluticasone (propionate or furoate), mometasone (furoate), ciclesonide and dexamethasone (sodium). Parentheses indicate preferred salt or ester forms. Particular mention should be made of budesonide, beclomethasone and fluticasone, especially budesonide, beclomethasone dipropionate, fluticasone propionate and fluticasone furoate.

Different classes of bronchodilators target different receptors in the airways. Two commonly used classes are β₂-agonists and anticholinergics.

β₂-Adrenergic agonists (or "β₂-agonists") act upon the β₂-adrenoceptors which induces smooth muscle relaxation, resulting in dilation of the bronchial passages. They tend to be categorised by duration of action. Examples of long-acting β₂-agonists (LABAs) include formoterol (fumarate), salmeterol (xinafoate), indacaterol (maleate), bambuterol (hydrochloride), clenbuterol (hydrochloride), olodaterol (hydrochloride), carmoterol (hydrochloride), tulobuterol (hydrochloride) and vilanterol (triphenylacetate). Examples of short-acting β₂-agonists (SABA) are albuterol (sulfate) and terbutaline (sulfate). Particular mention should be made of formoterol, salmeterol, indacaterol and vilanterol, especially formoterol fumarate, salmeterol xinafoate, indacaterol maleate and vilanterol triphenylacetate.

Typically short-acting bronchodilators provide a rapid relief from acute bronchoconstriction (and are often called "rescue" or "reliever" medicines), whereas long-acting bronchodilators help control and prevent longer-term symptoms. However, some rapid-onset long-acting bronchodilators may be used as rescue medicines, such as formoterol (fumarate). Thus, a rescue medicine provides relief from acute bronchoconstriction. The rescue medicine is taken as-needed/prn (pro re nata). The rescue medicine may also be in the form of a combination product, e.g. ICS-formoterol (fumarate), typically budesonide-formoterol (fumarate) or beclomethasone (dipropionate)-formoterol (fumarate). Thus, the rescue medicine is preferably a SABA or a rapid-acting LABA, more preferably albuterol (sulfate) or formoterol (fumarate), and most preferably albuterol (sulfate).

Anticholinergics (or "antimuscarinics") block the neurotransmitter acetylcholine by selectively blocking its receptor in nerve cells. On topical application, anticholinergics act predominantly on the M₃ muscarinic receptors located in the airways to produce smooth muscle relaxation, thus producing a bronchodilatory effect. Examples of long-acting muscarinic antagonists (LAMAs) include tiotropium (bromide), oxitropium (bromide), aclidinium (bromide), umeclidinium (bromide), ipratropium (bromide) glycopyrronium (bromide), oxybutynin (hydrochloride or hydrobromide), tolterodine (tartrate), trospium (chloride), solifenacin (succinate), fesoterodine (fumarate) and darifenacin (hydrobromide). Particular mention should be made of tiotropium, aclidinium, umeclidinium and glycopyrronium, especially tiotropium bromide, aclidinium bromide, umeclidinium bromide and glycopyrronium bromide.

A number of approaches have been taken in preparing and formulating these medicaments for delivery by inhalation, such as via a dry powder inhaler (DPI), a pressurized metered dose inhaler (pMDI) or a nebulizer.

According to the GINA (Global Initiative for Asthma) Guidelines, a step-wise approach is taken to the treatment of asthma. At step 1, which represents a mild form of asthma, the patient is given an as needed SABA, such as albuterol sulfate. The patient may also be given an as-needed low-dose ICS-formoterol, or a low-dose ICS whenever the SABA is taken. At step 2, a regular low-dose ICS is given alongside the SABA, or an as-needed low-dose ICS-formoterol. At step 3, a LABA is added. At step 4, the doses are increased and at step 5, further add-on treatments are included such as an anticholinergic or a low-dose oral corticosteroid. Thus, the respective steps may be regarded as treatment regimens, which regimens are each configured according to the degree of acute severity of the respiratory disease.

COPD is a leading cause of death worldwide. It is a heterogeneous long-term disease comprising chronic bronchitis, emphysema and also involving the small airways. The pathological changes occurring in patients with COPD are predominantly localised to the airways, lung parenchyma and pulmonary vasculature. Phenotypically, these changes reduce the healthy ability of the lungs to absorb and expel gases.

Bronchitis is characterised by long-term inflammation of the bronchi. Common symptoms may include wheezing, shortness of breath, cough and expectoration of sputum, all of which are highly uncomfortable and detrimental to the patient's quality of life. Emphysema is also related to long-term bronchial inflammation, wherein the inflammatory response results in a breakdown of lung tissue and progressive narrowing of the airways. In time, the lung tissue loses its natural elasticity and becomes enlarged. As such, the efficacy with which gases are exchanged is reduced and respired air is often trapped within the lung. This results in localised hypoxia, and reduces the volume of oxygen being delivered into the patient's bloodstream, per inhalation. Patients therefore experience shortness of breath and instances of breathing difficulty.

Patients living with COPD experience a variety, if not all, of these symptoms on a daily basis. Their severity will be determined by a range of factors but most commonly will be correlated to the progression of the disease. These symptoms, independent of their severity, are indicative of stable COPD and this disease state is maintained and managed through the administration of a variety drugs. The treatments are variable, but often include inhaled bronchodilators, anticholinergic agents, long-acting and short-acting β₂-agonists and corticosteroids. The medicaments are often administered as a single therapy or as combination treatments.

Patients are categorised by the severity of their COPD using categories defined in the GOLD Guidelines (Global Initiative for Chronic Obstructive Lung Disease, Inc.). The categories are labelled A-D and the recommended first choice of treatment varies by category. Patient group A are recommended a short-acting muscarinic antagonist (SAMA) prn or a short-acting β₂-aginist (SABA) prn. Patient group B are recommended a long-acting muscarinic antagonist (LAMA) or a long-acting β₂-aginist (LABA). Patient group C are recommended an inhaled corticosteroid (ICS) + a LABA, or a LAMA. Patient group D are recommended an ICS + a LABA and/or a LAMA.

Patients suffering from respiratory diseases like asthma or COPD suffer from periodic exacerbations beyond the baseline day-to-day variations in their condition. An exacerbation is an acute worsening of respiratory symptoms that require additional therapy, i.e. a therapy going beyond their maintenance therapy.

For asthma, the additional therapy for a moderate exacerbation are repeated doses of SABA, oral corticosteroids and/or controlled flow oxygen (the latter of which requires hospitalization). A severe exacerbation adds an anticholinergic (typically ipratropium bromide), nebulized SABA or IV magnesium sulfate.

For COPD, the additional therapy for a moderate exacerbation are repeated doses of SABA, oral corticosteroids and/or antibiotics. A severe exacerbation adds controlled flow oxygen and/or respiratory support (both of which require hospitalization).

An exacerbation within the meaning of the present disclosure includes both moderate and severe exacerbations.

Provided is a system for providing an output for warning of a respiratory disease exacerbation in a subject. The system comprises a first inhaler for delivering a rescue medicament to the subject. The rescue medicament may be suitable for treating a worsening of respiratory symptoms, for example by effecting rapid dilation of the bronchi and bronchioles upon inhalation of the medicament. The first inhaler has a use determination system configured to determine a rescue inhalation performed by the subject using the first inhaler. The system optionally includes a second inhaler for delivering a maintenance medicament to the subject during a routine inhalation. A sensor system is configured to measure a parameter relating to airflow during the rescue inhalation and/or during the routine inhalation, when the second inhaler is included in the system.

The rescue medicament is as defined hereinabove and is typically a SABA or a rapid-onset LABA, such as formoterol (fumarate). The rescue medicine may also be in the form of a combination product, e.g. ICS-formoterol (fumarate), typically budesonide-formoterol (fumarate). Such an approach is termed "MART" (maintenance and rescue therapy). However, the presence of a rescue medicine indicates that it is a first inhaler within the meaning of the present disclosure since the presence of the rescue medicament is determinative in the providing of the output. It therefore covers both a rescue medicament and a combination rescue and maintenance medicament. In contrast, the second inhaler, when present, is only used for the maintenance aspect of the therapy and not for rescue purposes. The key difference is that the first inhaler may be used as-needed, whereas the second inhaler is intended for use at regular, pre-defined times.

In a non-limiting example, the first inhaler is configured to deliver a rescue medicament selected from albuterol (sulfate), formoterol (fumarate), budesonide combined with formoterol (fumarate), beclomethasone (dipropionate) combined with albuterol (sulfate), and fluticasone (propionate or furoate) combined with albuterol (sulfate).

Alternatively or additionally, the second inhaler, when included in the system, is configured to deliver a maintenance medicament selected from budesonide, beclomethasone (dipropionate), fluticasone (propionate or furoate), and salmeterol (xinafoate) combined with fluticasone (propionate or furoate).

The system further comprises a processing module configured to monitor a frequency of the determined rescue inhalations. The processing module also monitors the parameter as a function of time. The processing module is further configured to provide the output for warning of the respiratory disease exacerbation if the parameter as a function of time is indicative of the lung condition of the subject deteriorating over a first time period, and the frequency of the determined rescue inhalations is higher during a second time period than during the first time period, the second time period being subsequent to the first time period.

Further provided is a method for providing an output for warning of a respiratory disease exacerbation in a subject. Any preferred embodiments discussed in respect of the system may be applied to the methods, and vice versa.

Attempts have been made to assess the risk of an impending respiratory disease exacerbation, such as an asthma or COPD exacerbation, by monitoring various subject-related and environmental factors. Challenges have been encountered concerning which factors should be taken into account, and which neglected. Neglecting factors which only have a minimal or negligible influence on the risk determination may enable determination of the risk more efficiently, for example using less computational resources, such as processing resources, battery power, memory requirements, etc. Of greater importance is the requirement to improve the accuracy with which an impending respiratory disease exacerbation may be determined. A more accurate risk determination may facilitate a more effective warning system so that the appropriate clinical intervention may be delivered to the subject.

The present inventors have found, from detailed analysis of patterns of inhaler use by subjects participating in clinical studies, which will be explained in more detail herein below, that lung condition deterioration as indicated by change in the parameter tends to be followed in time by increased frequency of rescue medicament usage prior to an exacerbation. This observation is utilized in the present system such that warning of an impending respiratory disease exacerbation may be made more reliable.

It is noted that the deterioration in the lung condition of the subject, as indicated by the change in the parameter as a function of time, may continue during the second time period.

Thus, in a non-limiting example, the processing module is configured to provide the output for warning of a respiratory disease exacerbation if the parameter as a function of time is indicative of the lung condition of the subject deteriorating over the first time period and the second time period, and the frequency of the determined rescue inhalations is higher during the second time period than during the first time period.

The frequency of the determined first/rescue inhaler uses may, for example, correspond to the number of rescue inhaler uses per day, in other words the number of daily rescue inhaler uses.

The first time period may be selected according to the time required to gather parameter versus time data of suitable diagnostic value. A sample period which is too short may not permit sufficient inhalation data to be collected for reliable exacerbation prediction, whilst a sample period which is too long may have an averaging effect which renders shorter term trends which are of diagnostic or predictive significance less distinguishable. The first time period may be, for example, 2 to 60 days, such as 5 to 55 days.

Similar considerations are applicable to selection of the second time period. The second time period may be, for example, 2 to 70 days, such as 5 to 65 days.

In an embodiment, the system comprises a user interface, and the processing module is configured to control the user interface to issue an exacerbation warning based on the output for warning of a respiratory disease exacerbation being provided by the processing module.

The user interface may, for example, be configured to enable user-inputting of an indication of a status of the respiratory disease being experienced by the subject. In this non-limiting example, the processing module is configured to control the user interface to issue a prompt to input the indication based on the output for warning of a respiratory disease exacerbation being provided by the processing module.

In this manner, the inhaler usage data may be supplemented by input from the subject. The user-inputted indication may provide information which confirms or validates the warning output based on inhaler usage data.

Moreover, this approach to prompting user-inputting of the indication may reduce the burden on the subject as compared to, for example, the scenario in which the user is routinely prompted to input the indication, irrespective of their inhaler use. This, in turn, may render it more likely that the subject will input the indication when prompted to do so. Thus, improved monitoring of the subject's respiratory disease may be enabled by this exemplary system.

The processing module may, for instance, be configured to control the user interface to issue an exacerbation warning, as well as the prompt to input the indication, based on the output being provided by the processing module.

The user interface may thus be configured to enable user-inputting of the indication, and issuing of the prompt and/or exacerbation warning.

The user interface may, for example, comprise a first user interface configured to enable using-inputting of the indication, and a second user interface configured to, when controlled by the processing module, output the prompt.

The first and second user interface may, for instance, be included in the same user device.

In a non-limiting example, the user interface comprises a touchscreen. In such an example, the second user interface comprises the display of the touchscreen, and the first user interface comprises the touch inputting system of the touchscreen. Such a touchscreen enables facile user-inputting and prompting, and is thus particularly beneficial in the scenario in which the subject is suffering from worsening symptoms, as indicated by the parameter and the frequency of rescue medicament usage.

As an alternative or in addition to the prompt being issued via the touchscreen, the second user interface may comprise a loudspeaker for issuing, when controlled by the processing module, an audible prompt.

In an embodiment, the user interface, e.g. the first user interface, is configured to provide a plurality of user-selectable respiratory disease status options. In this case, the indication is defined by user-selection of at least one of the status options.

The user interface may, for example, prompt the user or subject to provide the indication via a pop-up notification link to complete a short questionnaire.

In a non-limiting example, the user interface displays a questionnaire comprising questions whose answers correspond to the indication. The user, e.g. the subject or his/her health care provider, may input the answers to the questions using the user interface.

In an embodiment, the system comprises a memory, for example a memory included in the processing module, for storing each indication inputted via the user interface. The indication may be subsequently retrieved, for example to support a dialogue between the subject and his/her healthcare provider. In this manner, the subject's recollection of a previous status of their respiratory disease need not be relied upon for the purposes of the dialogue.

The questionnaire may be relatively short, i.e. with relatively few questions, in order to minimize burden on the subject. The number and nature of the questions may nevertheless be such as to ensure that the indication enables the clinical condition of the subject, e.g. including the likelihood of the subject experiencing an exacerbation, to be reliably assessed.

Particular mention is made of inputting the indication in the form of a six-point/six-question questionnaire because the requirement for sufficient clinical information is balanced with avoiding placing too much burden on the subject, particularly as he/she may be suffering from worsening symptoms, as indicated by the parameter and the frequency of rescue medicament usage.

More generally, the object of the questionnaire is to ascertain a contemporaneous or relatively recent (e.g. within the past 24 hours) indication in order to obtain "in the moment" understanding of the subject's well-being (in respect of their respiratory disease) with a few timely questions which are relatively quickly answered. The questionnaire may be translated into the local language of the subject.

Conventional control questionnaires, and especially the most established being ACQ/T (Asthma Control Questionnaire / Test) in asthma, or CAT (COPD Assessment Test) in COPD tend to focus on patient recall of symptoms in the past. Recall bias, and a focus on the past instead of the present is likely to negatively influence their value for the purposes of predictive analysis.

The following is provided by way of non-limiting example of such a questionnaire. The subject may select from the following status options for each question: All of the time (5); Most of the time (4); Some of the time (3); A little (2); None (1).

| | |
|---|---|
| 1. | How 'often are you experiencing', or 'Rate your' shortness of breath? |
| 2. | How 'often are you experiencing', or 'Rate your' coughing? |
| 3. | How 'often are you experiencing', or 'Rate your' wheezing? |
| 4. | How 'often are you experiencing', or 'Rate your' chest tightness? |
| 5. | How 'often are you experiencing', or 'Rate your' night symptoms/affecting sleep? |
| 6. | How 'often are you experiencing', or 'Rate your' limitation at work, school or home? |

An alternative example questionnaire is also provided:

| | |
|---|---|
| 1. | Are you having more respiratory symptoms than usual (Y/N)? If yes: |
| 2. | More chest tightness or shortness of breath (Y/N)? |
| 3. | More cough (Y/N)? |
| 4. | More wheezing (Y/N)? |
| 5. | Is it affecting your sleep (Y/N)? |
| 6. | Is it limiting your activities at home/work/school (Y/N)? |

Still another example questionnaire is also provided:

| | |
|---|---|
| 1. | Are you having more: |
| | chest tightness or shortness of breath? (Y/N) |
| | cough? (Y/N) |
| | wheezing? (Y/N) |
| 2. | Are you sleeping well? (Y/N) |
| 3. | Are you limiting your daily activities in any way? (Y/N) |
| 4. | Have you had an infection or allergen (e.g. cat, pollen) exposure? (Y/N) |

Yet another example questionnaire is also provided:

| | |
|---|---|
| 1. | Are you having: |
| | More chest tightness or shortness of breath? (Y/N) |
| | More cough? (Y/N) |
| | More wheezing? (Y/N) |
| 2. | Are you sleeping well? (Y/N) |
| 3. | Are you limiting your activities at home/work/school? (Y/N) |
| 4. | Have you had an infection? (Y/N) |
| | If yes, did you take any antibiotics and/or steroids? (Y/N) |
| 5. | Have you had an allergen (e.g. cat, pollen) exposure recently? (Y/N) |
| 6. | (Optional) What is your most recent hospital anxiety and depression scale (HADS) score? |

The answers to the questions may, for example, be used to calculate a score, which score is included in, or corresponds to, the indication of the status of the respiratory disease being experienced by the subject.

More generally, a memory included in the system is, in an embodiment, configured to store the indication, e.g. the answers to the questionnaire and/or the score, inputted via the user interface. Thus, the stored indication can be later retrieved for the patient-to-healthcare provider dialogue.

In an embodiment, the user interface is configured to provide the status options in the form of selectable icons, e.g. emoji-type icons, checkboxes, a slider, and/or a dial. In this way, the user interface may provide a straightforward and intuitive way of inputting the indication of the status of the respiratory disease being experienced by the subject. Such intuitive inputting may be particularly advantageous when the subject himself/herself is inputting the indication, since the relatively facile user-input may be minimally hampered by any worsening of the subject's respiratory disease.

Any suitable user interface may be employed for the purpose of enabling user-input of the indication of the status of the respiratory disease being experienced, e.g. subjectively, by the subject. For example, the user interface may comprise or consist of a user interface of a user device. The user device may be, for example, a personal computer, a tablet computer, and/or a smart phone. When the user device is a smart phone, the user interface may, for instance, correspond to the touchscreen of the smart phone, as previously described.

In some non-limiting examples, the system may be further configured such that the indication can be inputted via the user interface when the user opts to so input the indication. Thus, the user, e.g. the subject, need not wait for the prompt in order to input the indication.

Alternatively or additionally, the processing module may be configured to issue the prompt based on no flags, including the output, indicating worsening of the subject's condition are triggered during a predetermined time period, e.g. 7 days.

This may assist to a) ensure that there are no symptoms that the patient is having that the use determination system (use and/or inhalation parameter) is missing; and/or b) to capture if a patient is well (e.g. all 'no' answers to the above-described questionnaire) and that the indication and the rescue inhaler use and inhalation parameter data are thus aligned with each other; and/or c) as a way to capture whether and when the patient is recovering.

The processing module may include a general purpose processor, a special purpose processor, a DSP, a microcontroller, an integrated circuit, and/or the like that may be configured using hardware and/or software to perform the functions described herein for the processing module. The processing module may be included partially or entirely in the inhaler, a user device, and/or a server.

The processing module may include a power supply, memory, and/or a battery.

In a non-limiting example, the processing module is at least partly included in a first processing module included in the user device. In other non-limiting examples, the processing module is not included in a user device. The processing module (or at least part of the processing module) may, for example, be provided in a server, e.g. a remote server. For example, the processing module may be implemented on any combination of the first and/or second inhaler, the user device, and/or a remote server. As such, any combination of the functions or processing described with reference to the processing module may be performed by a processing module residing on the first and/or second inhaler, the user device, and/or a server. For instance, the use determination system residing on the first inhaler may capture usage information at the first inhaler (e.g. such as a use or manipulation of the inhaler by the user (such as the opening of a mouthpiece cover and/or the actuation of a switch) and/or the parameter relating to airflow during a use of the inhaler), while the processing module residing on any combination of the inhaler, the user device, and/or server may determine inhalation parameters based on the parameter relating to airflow during a use of the inhaler and/or determine notifications, such as the above-described output, associated with the rescue inhaler uses and/or inhalation parameters.

The use determination system may, for example, comprise a sensor for detecting an inhalation of the rescue medicament performed by the subject and/or a mechanical switch configured to be actuated prior to, during, or after use of the first inhaler. In this way, the use determination system enables recording of each use, or attempted use, of the first inhaler.

The first inhaler may, for instance, comprise a mouthpiece through which the user performs the inhalation, and a mouthpiece cover. In such an example, the mechanical switch may be configured to be actuated when the mouthpiece cover is moved to expose the mouthpiece.

In a non-limiting example, the first inhaler comprises a medicament reservoir, and a dose metering assembly configured to meter a dose of the medicament from the reservoir. In this particular example, the use determination system is configured to register the metering of the dose by the dose metering assembly. Each metering is thereby indicative of the rescue inhalation performed by the subject using the first inhaler.

In certain examples, the use determination system employs the sensor in combination with the mechanical switch in order to determine the parameter relating to airflow during a use of the first inhaler by the subject.

Such a sensor may, for example, comprise a pressure sensor, such as an absolute or differential pressure sensor.

The sensor system is configured to sense the parameter during rescue inhalations of the rescue medicament performed by the subject using the first inhaler and/or during routine inhalations of the maintenance medicament performed by the subject using the second inhaler when it is included in the system.

The parameter relating to airflow during the inhalation may act as a proxy for the lung condition of the subject.

Any suitable parameter relating to airflow can be considered. In a non-limiting example, the parameter is at least one of a peak inhalation flow, an inhalation volume, a time to peak inhalation flow, and an inhalation duration. The parameter is preferably an inhalation volume and/or a peak inhalation flow.

Lung condition deterioration as measured, for example, via the inhaled volume may provide effective early warning of an exacerbation. Change in inhalation volume, e.g. over time, has been observed to occur earlier and/or to a greater extent than changes in other parameter types, such a peak inhalation flow.

The use determination system may, for example, also comprise a pressure sensor which may be the same as or different from that included in the sensor system.

Further provided is a method for providing an output for warning of a respiratory disease exacerbation in a subject. The method comprises monitoring a frequency of rescue inhalations using a rescue inhaler, and monitoring a parameter relating to airflow as a function of time, the parameter being sensed during the rescue inhalations and/or during routine inhalations of a maintenance medicament performed by the subject. The output for warning of the respiratory disease exacerbation is provided if the parameter as a function of time is indicative of the lung condition of the subject deteriorating over a first time period, and the frequency of rescue inhalations is higher during a second time period than during the first time period, the second time period being subsequent to the first time period.

In an embodiment, the method further comprises controlling a user interface to issue an exacerbation warning based on the output for warning of the respiratory disease exacerbation being provided.

Alternatively or additionally, the method comprises controlling a user interface, which user interface is configured to enable user-inputting of an indication of a status of a respiratory disease being experienced by the subject, to issue a prompt to input the indication based on the output being provided.

The method may, for instance, further comprise storing the indication inputted via the user interface in a memory. The stored indication may be retrieved for a dialogue between the subject and their healthcare provider, as previously described.

A computer program is also provided, which computer program comprises computer program code which is adapted, when the computer program is run on a computer, to implement the method. In an example, the computer code may reside partially or entirely on a user device (e.g. as a mobile application residing on the user device).

The embodiments described herein for the system are applicable to the method and the computer program. Moreover, the embodiments described for the method and computer program are applicable to the system.

Further provided is a method for treating a respiratory disease exacerbation in a subject, the method comprising: performing the method as defined above; and treating said respiratory disease based on the output for warning of the respiratory disease exacerbation being provided.

The treatment may comprise modifying an existing treatment. The existing treatment may comprise a first treatment regimen, and the modifying the existing treatment of the respiratory disease may comprise changing from the first treatment regimen to a second treatment regimen based on the output being provided, wherein the second treatment regimen is configured for higher risk of a respiratory disease exacerbation than the first treatment regimen.

The output determination may facilitate a more effective warning system so that the appropriate clinical intervention may be delivered to the subject. The more reliably based exacerbation warning may have the potential to guide intervention for a subject at acute risk. In particular, the intervention may include implementing the second treatment regimen. This may, for example, involve progressing the subject to a higher step specified in the GINA or GOLD guidelines. Such preemptive intervention may mean that the subject need not proceed to suffer the exacerbation, and be subjected to the associated risks, in order for the progression to the second treatment regimen to be justified.

In an embodiment, the second treatment regimen comprises administering a biologics medication to the subject. The relatively high cost of biologics means that stepping up the subject's treatment to include administering of a biologics medication tends to require careful consideration and justification. The systems and methods according to the present disclosure may provide a reliable metric, in terms of the output, to justify administering of a biologics medication. For example, should a number of times that the output is provided reach a predetermined number, the administering of the biologics medication may be quantitatively justified, and the biologics medication may be administered accordingly.

More generally, the biologics medication may comprise one or more of omalizumab, mepolizumab, reslizumab, benralizumab, and dupilumab.

Modifying the existing treatment of the respiratory disease may comprise changing from the first treatment regimen to a third treatment regimen based on the output not being provided for a predetermined period of time. The third treatment regimen may be configured for lower risk of a respiratory disease exacerbation than the first treatment regimen.

In other words, during the predetermined period of time in which the subject is monitored, such as 6 to 12 months, deterioration in the subject's lung condition indicated by the change in the parameter over time may not occur, or if it does it may not be followed in time by increased frequency of rescue inhaler use. Thus, the output is not provided during the predetermined period of time. The third treatment regimen may accordingly be configured for lower risk of a respiratory disease exacerbation than the first treatment regimen.

In this case, enhanced accuracy of the probability determination may be used as guidance to justify downgrading or even removal of an existing treatment regimen. In particular, the subject may be moved from the first treatment regimen onto the third treatment regimen which is configured for lower risk of respiratory disease exacerbation than the first treatment regimen. This may, for example, involve progressing the subject to a lower step specified in the GINA or GOLD guidelines.

Thus, the system and methods according to the present disclosure may be used to monitor subject recovery, and may, for instance, be used to justify withdrawal of oral steroids or other medication. This may assist to lessen the risk of hospital/healthcare setting re-admission.

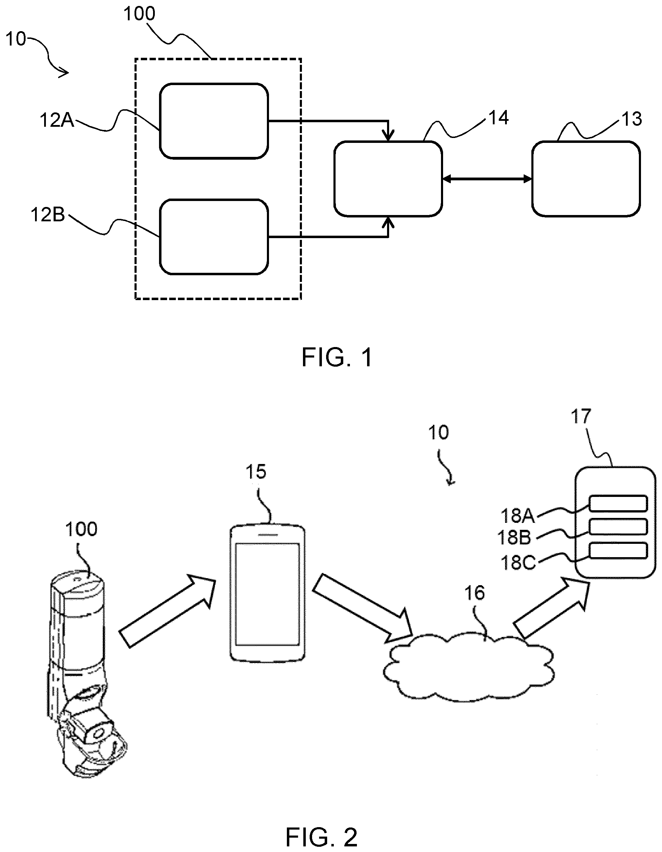

Fig. 1 shows a block diagram of a system 10 according to an embodiment. The system 10 comprises a first inhaler 100 and a processing module 14. The first inhaler 100 may be used to deliver a rescue medicament, such as a SABA, to the subject. The SABA may include, for example, albuterol. The first inhaler 100 may include a sensor system 12A and a use determination system 12B.

The system 10 may, for example, be alternatively termed "an inhaler assembly".

The first inhaler may, for example, be alternatively termed "a rescue inhaler".

Whilst not visible in Fig. 1, the system may also include a second inhaler for delivering a maintenance medicament to the subject. The second inhaler may, for example, be alternatively termed "a maintenance inhaler" or "a controller inhaler".

A rescue inhalation is determined by the use determination system 12B included in the first inhaler 100.

A sensor system 12A may be configured to measure the parameter. The sensor system 12A may, for example, comprise one or more sensors, such as one or more pressure sensors, temperature sensors, humidity sensors, orientation sensors, acoustic sensors, and/or optical sensors. The pressure sensor(s) may include a barometric pressure sensor (e.g. an atmospheric pressure sensor), a differential pressure sensor, an absolute pressure sensor, and/or the like. The sensors may employ microelectromechanical systems (MEMS) and/or nanoelectromechanical systems (NEMS) technology.

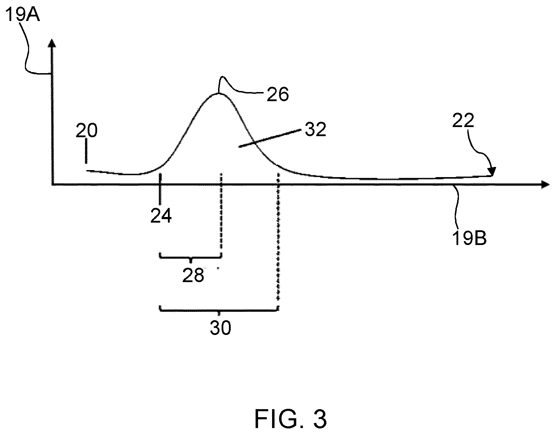

A pressure sensor may be particularly suitable for measuring the parameter, since the airflow during inhalation by the subject may be monitored by measuring the associated pressure changes. As will be explained in greater detail with reference to Figs. 3 and 13-17, a pressure sensor may be, for instance, located within or placed in fluid communication with a flow pathway through which air and the medicament is drawn by the subject during inhalation. Alternative ways of measuring the parameter, such as via a suitable flow sensor, will also be apparent to the skilled person.

Alternatively or additionally, the sensor system 12A may comprise a differential pressure sensor. The differential pressure sensor may, for instance, comprise a dual port type sensor for measuring a pressure difference across a section of the air passage through which the subject inhales. A single port gauge type sensor may alternatively be used. The latter operates by measuring the difference in pressure in the air passage during inhalation and when there is no flow. The difference in the readings corresponds to the pressure drop associated with inhalation.

Whilst not shown in Fig. 1, the system 10 may further comprise a second inhaler for delivering a maintenance medicament to the subject during a routine inhalation. The second inhaler may include a sensor system 12A and/or a use determination system 12B that is distinct from the sensor system 12A and/or the use determination system 12B of the first inhaler 100. The sensor system 12A of the second inhaler may be configured to measure the parameter during the routine inhalation. For example, the sensor system 12A may include a further pressure sensor, such as a further microelectromechanical system pressure sensor or a further nanoelectromechanical system pressure sensor, in order to measure the parameter during maintenance medicament inhalation.

In this manner, inhalation of either or both the rescue and the maintenance medicaments may be used to gather information relating to the subject's lung condition, e.g. the subject's lung function and/or lung health.

When both the first and second inhalers are used, the accuracy with which an impending exacerbation can be predicted may be improved by the additional inhalation data supplied by monitoring both routine and rescue medicament inhalations.

Each inhalation may be associated with a decrease in the pressure in the airflow channel relative to when no inhalation is taking place. The point at which the pressure is at its lowest may correspond to the peak inhalation flow. The sensor system 12A may detect this point in the inhalation. The peak inhalation flow may vary from inhalation to another inhalation, and may depend on the clinical condition of the subject. Lower peak inhalation flows may, for example, be recorded when the subject is approaching an exacerbation.

The pressure change associated with each inhalation may alternatively or additionally be used to determine an inhalation volume. This may be achieved by, for example, using the pressure change during the inhalation measured by the sensor system 12A to first determine the flow rate over the time of the inhalation, from which the total inhaled volume may be derived. Lower inhalation volumes may be recorded when, for instance, the subject is approaching an exacerbation, since the subject's capacity to inhale may be diminished.

The pressure change associated with each inhalation may alternatively or additionally be used to determine an inhalation duration. The time may be recorded, for example, from the first decrease in pressure measured by the pressure sensor 12A, coinciding with the start of the inhalation, to the pressure returning to a pressure corresponding to no inhalation taking place. Lower inhalation durations may be recorded when, for instance, the subject is approaching an exacerbation, since the subject's capacity for inhaling for longer may be diminished.

In an embodiment, the parameter includes the time to peak inhalation flow, e.g. as an alternative or in addition to the peak inhalation flow, the inhalation volume and/or the inhalation duration. This time to peak inhalation flow parameter may be recorded, for example, from the first decrease in pressure measured by the sensor system 12A, coinciding with the start of the inhalation, to the pressure reaching a minimum value corresponding to peak flow. A subject who is at greater risk of an exacerbation may take a longer time to achieve peak inhalation flow.

In a non-limiting example, the first and/or second inhalers may be configured such that, for a normal inhalation, the respective medicament is dispensed during approximately 0.5 s following the start of the inhalation. A subject's inhalation only reaching peak inhalation flow after the 0.5 s has elapsed, such as after approximately 1.5 s, may be partially indicative of an impending exacerbation.

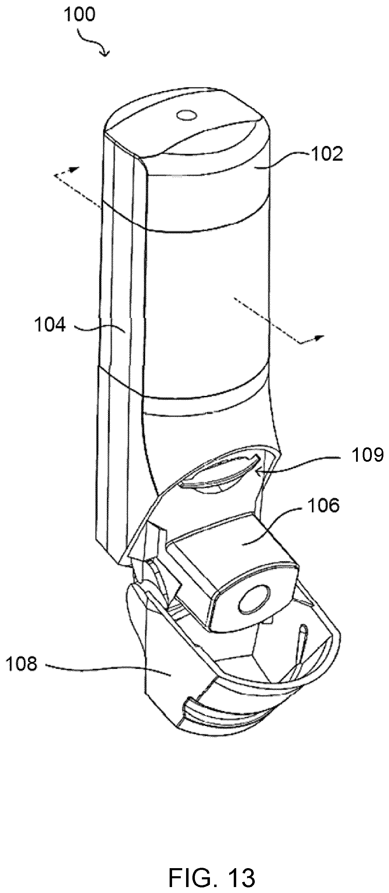

The use determination system 12B is configured to register inhalation(s) by the subject (e.g. each rescue inhalation by the subject when the inhaler is a rescue inhaler, or each maintenance inhalation by the subject when the inhaler is a maintenance inhaler). In a non-limiting example, the first inhaler 100 may comprise a medicament reservoir (not shown in Fig. 1), and a dose metering assembly (not shown in Fig. 1) configured to meter a dose of the rescue medicament from the reservoir. The use determination system 12B may be configured to register the metering of the dose by the dose metering assembly, each metering being thereby indicative of the rescue inhalation performed by the subject using the first inhaler 100. Accordingly, the inhaler 100 may be configured to monitor the number of rescue inhalations of the rescue medicament, since the dose must be metered via the dose metering assembly before being inhaled by the subject. One non-limiting example of the metering arrangement will be explained in greater detail with reference to Figs. 13-16.

Alternatively or additionally, the use determination system 12B may register each inhalation in different manners and/or based on additional or alternative feedback that are apparent to the skilled person. For example, the use determination system 12B may be configured to register an inhalation by the subject when the feedback from the sensor system 12A indicates that an inhalation by the user has occurred (e.g. when a pressure measurement or flow rate exceeds a predefined threshold associated with a successful inhalation). Further, in some examples, the use determination system 12B may be configured to register an inhalation when a switch of the first inhaler 100 or a user input of an external device (e.g. touchscreen of a smartphone) is manually actuated by the subject prior to, during or after inhalation.

A sensor (e.g. a pressure sensor) may, for example, be included in the use determination system 12B in order to register each inhalation. In such an example, the use determination system 12B and the sensor system 12A may employ respective sensors (e.g. pressure sensors), or a common sensor (e.g. a common pressure sensor) which is configured to fulfil both use determination and inhalation parameter sensing functions.

When a sensor is included in the use determination system 12B, the sensor may, for instance, be used to confirm that, or assess the degree to which, a dose metered via the dose metering assembly is inhaled by the user, as will be described in greater detail with reference to FIGs. 3 and 13-16.

In an embodiment, the sensor system 12A and/or the use determination system 12B includes an acoustic sensor. The acoustic sensor in this embodiment is configured to sense a noise generated when the subject inhales through the respective inhaler. The acoustic sensor may include, for example, a microphone.

In a non-limiting example, the respective inhaler may comprise a capsule which is arranged to spin when the subject inhales though the device; the spinning of the capsule generating the noise for detection by the acoustic sensor. The spinning of the capsule may thus provide a suitably interpretable noise, e.g. rattle, for deriving use and/or inhalation parameter data.

An algorithm may, for example, be used to interpret the acoustic data in order to determine use data (when the acoustic sensor is included in the use determination system 12B) and/or the parameter relating to airflow during the inhalation (when the acoustic sensor is included in the sensor system 12A).

For instance, an algorithm as described by Colthorpe et al. in "Adding Electronics to the Breezhaler: Satisfying the Needs of Patients" (Respiratory Drug Delivery 2018; page 71-79) may be used. Once the generated sound is detected, the algorithm may process the raw acoustic data to generate the use and/or inhalation parameter data.

The system 10 further comprises a user interface 13. The user interface 13 may, for instance, be controlled to issue an exacerbation warning and/or a prompt for the subject to input an indication of the status of the respiratory disease being experienced by the subject, as previously described. Any suitable user interface 13 may be employed for this purpose, such as the touch screen of a smart phone.

The processing module 14 included in the system 10 monitors a frequency of the determined rescue inhalations, and monitors the inhalation parameter as a function of time. As schematically shown in Fig. 1 by the arrows between the sensor system 12A and the processing module 14, and between the use determination system 12B and the processing module 14, the processing module 14 may receive the determined rescue inhalation and parameter data from the use determination system 12B and the sensor system 12A respectively.

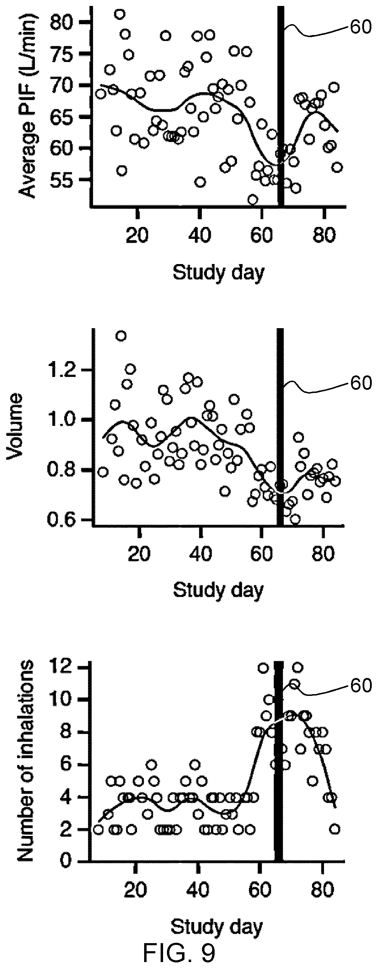

The processing module is further configured to provide the output for warning of a respiratory disease exacerbation if the parameter as a function of time is indicative of the lung condition of the subject deteriorating over a first time period, and the frequency of the determined rescue inhalations is higher during a second time period than during the first time period, the second time period being subsequent to the first time period, as will be discussed in greater detail with reference to Figs. 9-12.

In a non-limiting example, the processing module 14 may be provided separately from the respective first and/or second inhaler(s), in which case the processing module 14 receives the deterimed rescue inhalations and parameter data transmitted to it from the sensor system 12A and the use determination system 12B of the first and/or second inhalers. By processing the data in such an external processing unit, such as in the processing unit of an external device, the battery life of the inhaler may be advantageously preserved.

In an alternative non-limiting example, the processing module 14 may be an integral part of the first and/or second inhaler, for example contained within a main housing or top cap (not shown in Fig. 1) of the first and/or second inhaler. In such an example, connectivity to an external device need not be relied upon, since the output may be provided via processing implemented exclusively within the first and/or second inhaler. The first and/or second inhaler may, for instance, include a suitable user interface, such as a light or lights, screen, loudspeaker, etc., for providing the exacerbation warning to the subject based on the processing module 14 providing the output. The first and/or second inhaler may thus, for example, prompt the subject to take preemptive steps to mitigate or remove the risk of an exacerbation.

It may also be contemplated that some of the functions of the processing module 14 may be performed by an internal processing unit included in the first and/or second inhaler and other functions of the processing module, such as providing the output, may be performed by the external processing unit.

More generally, the system 10 may include, for example, a communication module (not shown in Fig. 1) configured to communicate the exacerbation warning, based on the output provided by the processing module 14, to the subject and/or a healthcare provider, such as a clinician. The subject and/or the clinician may then take appropriate steps to mitigate the risk of the subject experiencing an exacerbation. When, for instance, a smart phone processing unit is included in the processing module, the communication functions of the smart phone, such as SMS, email, Bluetooth^{®}, etc., may be employed to communicate the exacerbation warning based on the output of the processing module to the healthcare provider.

Fig. 2 shows a non-limiting example of a system 10 for providing an output for warning of a respiratory disease exacerbation in a subject. An exacerbation warning based on the output may be provided to the subject, caregiver and/or healthcare provider.

The example system 10 includes the first inhaler 100, an external device 15 (e.g. a mobile device), a public and/or private network 16 (e.g. the Internet, a cloud network, etc.), and a personal data storage device 17. The external device 15 may, for example, include a smart phone, a personal computer, a laptop, a wireless-capable media device, a media streaming device, a tablet device, a wearable device, a Wi-Fi or wireless-communication-capable television, or any other suitable Internet Protocol-enabled device. For example, the external device 15 may be configured to transmit and/or receive RF signals via a Wi-Fi communication link, a Wi-MAX communications link, a Bluetooth^{®} or Bluetooth^{®} Smart communications link, a near field communication (NFC) link, a cellular communications link, a television white space (TVWS) communication link, or any combination thereof. The external device 15 may transfer data through the public and/or private network 16 to the personal data storage device 17.

The first inhaler 100 may include a communication circuit, such as a Bluetooth^{®} radio, for transferring data to the external device 15. The data may include the abovementioned rescue inhalation and inhalation parameter data.

The first inhaler 100 may also, for example, receive data from the external device 15, such as, for example, program instructions, operating system changes, dosage information, alerts or notifications, acknowledgments, etc.

The external device 15 may include at least part of the processing module 14, and thereby process and analyze the rescue inhalation and parameter data. For example, the external device 15 may process the data such as to provide the output for warning of the respiratory disease exacerbation, as represented by block 18A, and provide such information to the personal data storage device 17 for remote storage thereon.

In some non-limiting examples, the external device 15 may also process the data to identify no inhalation events, low inhalations events, good inhalation events, excessive inhalation events and/or exhalation events, as represented by block 18B. The external device 15 may also process the data to identify underuse events, overuse events and optimal use events, as represented by block 18C. The external device 15 may, for instance, process the data to estimate the number of doses delivered and/or remaining and to identify error conditions, such as those associated with a timestamp error flag indicative of failure of the subject to inhale a dose of the medicament which has been metered by the dose metering assembly. The external device 15 may include a display and software for visually presenting the usage parameters through a GUI on the display. The usage parameters may be stored as personalized data that may be stored for predicting future risk of exacerbations based on real-time data.

Although illustrated as being stored on the personal data storage device 17, in some examples, all or a portion of the processing of the output for warning of a respiratory disease exacerbation, as represented by block 18A, the no inhalation events, low inhalations events, good inhalation events, excessive inhalation events and/or exhalation events, as represented by block 18B, and/or the underuse events, overuse events and optimal use events, as represented by block 18C, may be stored on the external device 15.

Fig. 3 shows a graph of flow rate 19A versus time 19B during use of an inhaler 100 according to a non-limiting example. The use determination system 12B in this example comprises a mechanically operated switch in the form of a switch which is actuated when a mouthpiece cover of the inhaler 100 is opened. The mouthpiece cover is opened at point 20 on the graph. In this example, the use determination system 12B and sensor system 12A both comprise a pressure sensor.

When the mouthpiece cover is opened, the use determination system 12B is woken out of an energy-saving sleep mode, and a new inhalation event is registered. The inhalation event is also assigned an open time corresponding to how much time, for example in milliseconds, elapses since the inhaler 100 wakes from the sleep mode. Point 22 corresponds to the cap closing or 60 seconds having elapsed since point 20. At point 22, detection ceases.

Once the mouthpiece cover is open, the use determination system 12B looks for a change in the air pressure, as detected using the pressure sensor. The start of the air pressure change is registered as the inhale event time 24. The point at which the air pressure change is greatest corresponds to the peak inhalation flow 26. The sensor system 12A records the peak inhalation flow 26 as a flow of air, measured in units of 100 mL per minute, which flow of air is transformed from the air pressure change. Thus, in this non-limiting example, the parameter includes a numerical value of the peak inhalation flow in units of 100 mL per minute.

The time to peak inhalation flow 28 corresponds to the time taken in milliseconds for the peak inhalation flow 26 to be reached. The inhalation duration 30 corresponds to the duration of the entire inhalation in milliseconds. The area under the graph 32 corresponds to the inhalation volume in milliliters.

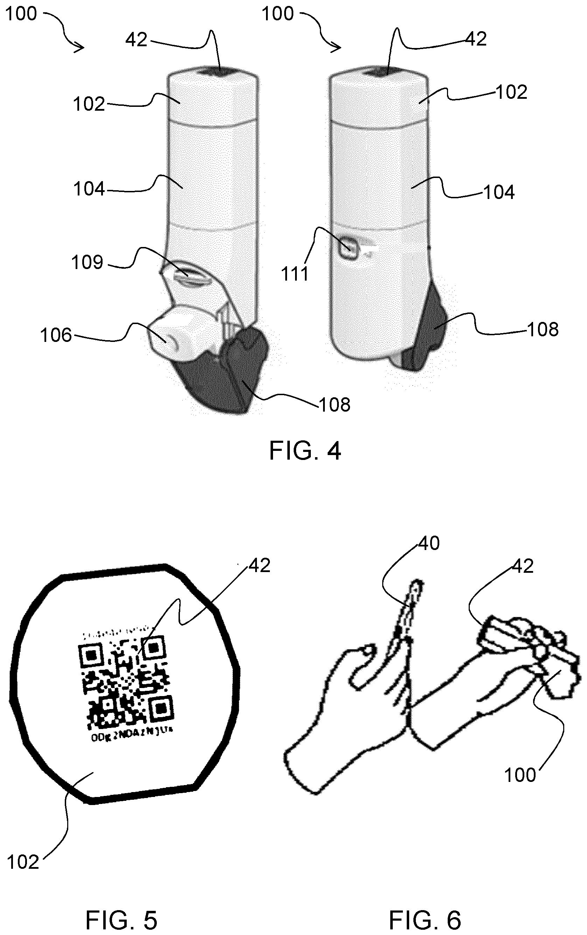

Fig. 4 shows front and rear views of the exterior of an inhaler 100, e.g. the first and/or second inhaler, according to a non-limiting example. The inhaler 100 comprises a top cap 102, a main housing 104, a mouthpiece 106, a mouthpiece cover 108, and an air vent 109. The mouthpiece cover 108 may be hinged to the main housing 104 so that it may open and close to expose the mouthpiece 106 and the air vent 109. The depicted inhaler 100 also comprises a mechanical dose counter 111, whose dose count may be used to check the number of doses remaining as determined by the processing module (on the basis of the total number of doses contained by the inhaler 100 prior to use and on the uses determined by the use determination system 12B).

In the non-limiting example shown in Fig. 4, the inhaler 100 has a barcode 42 printed thereon. The barcode 42 in this example is a quick reference (QR) code printed on the uppermost surface of the top cap 102. The use determination system 12B and/or the sensor system 12A may, for example, be located at least partly within the top cap 102, for example as components of an electronics module (not visible in Fig. 4). The electronics module of the inhaler 100 will be described in greater detail with reference to Figs. 13 to 16.

The QR code is more clearly visible in Fig. 5, which provides a view from directly above the top cap 102 of the inhaler 100 shown in Fig. 4. The QR code 42 may provide a facile way of pairing the respective inhaler 100 with the processing module 14, in examples in which a user device 40, comprising at least part of the processing module 14, also comprises a suitable optical reader, such as a camera, for reading the QR code. Fig. 6 shows a user pairing the inhaler 100 with the processing module 14 using the camera included in the user device 40, which in this particular example is a smart phone.

In other non-limiting examples, the processing module 14 may be paired with the respective inhaler 100 by, for example, manual entry of an alphanumerical key via a user interface 13 of the user device 40, e.g. a touchscreen.

Such a bar code 42, e.g. QR code, may comprise the identifier which is assigned to the respective medicament of the inhaler 100. Table A provides a non-limiting example of the identifiers included in the QR code 42 for various inhalers 100.

**Table A.**

| **Identifier in QR code** | **Brand of inhaler** | **Medicament** | **Dose strength (mcg)** | **Total dose count of inhaler prior to use** | **Medicament identification number** |
|---|---|---|---|---|---|
| <blank> | ProAir Digihaler | albuterol | 117 | 200 | AAA200 |
| AAA030 | ProAir Digihaler | albuterol | 117 | 30 | AAA030 |
| FSL060 | AirDuo Digihaler | fluticasone/ salmeterol | 55/14 | 60 | FSL060 |
| FSM060 | AirDuo Digihaler | fluticasone/ salmeterol | 113/14 | 60 | FSM060 |
| FSH060 | AirDuo Digihaler | fluticasone/ salmeterol | 232/14 | 60 | FSH060 |
| FPL060 | ArmonAir Digihaler | Fluticasone | 55 | 60 | FPL060 |
| FPM060 | ArmonAir Digihaler | Fluticasone | 113 | 60 | FPM060 |
| FPH060 | ArmonAir Digihaler | Fluticasone | 232 | 60 | FPH060 |

More generally, the processing module 14 may be configured to, e.g. following successful pairing of the processing module 14 with the respective inhaler 100, control the user interface 13 to notify the user that the above-described prompt may, at some point(s), be issued. For example, the user interface 13 may be controlled to issue the following message: "You may get sent a short questionnaire at any time, please just complete it truthfully."

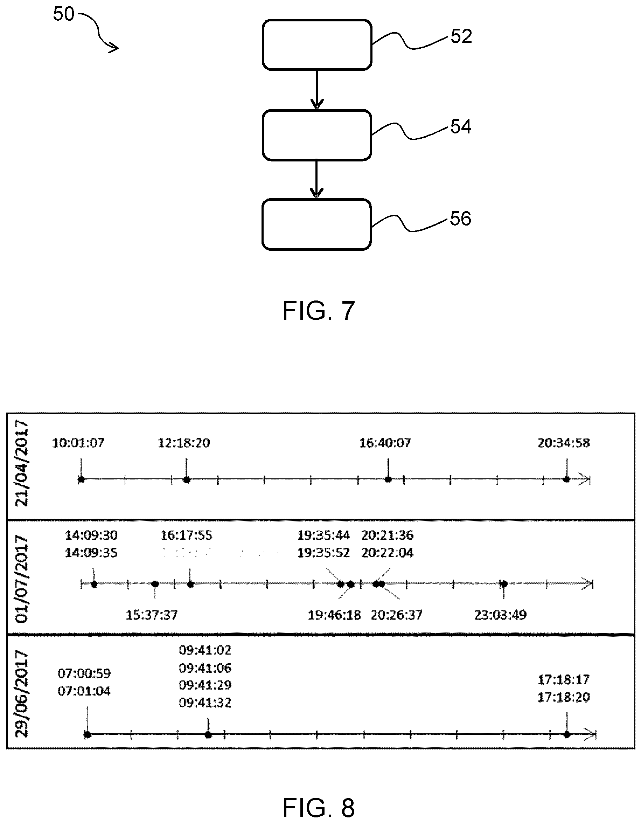

Fig. 7 provides a flowchart of a method 50 according to an example. The method 50 is for providing an output for warning of a respiratory disease exacerbation in a subject. The method 50 comprises monitoring 52 a frequency of rescue inhalations using a rescue inhaler, and monitoring 54 a parameter relating to airflow as a function of time, the parameter being sensed during the rescue inhalations and/or during routine inhalations of a maintenance medicament performed by the subject. The output for warning of the respiratory disease exacerbation is provided in step 56 if the parameter as a function of time is indicative of the lung condition of the subject deteriorating over a first time period, and the frequency of rescue inhalations is higher during a second time period than during the first time period, the second time period being subsequent to the first time period.

Whilst not shown in Fig. 7, the method 50 may further comprise controlling a user interface to issue an exacerbation warning based on the output for warning of the respiratory disease exacerbation being provided.

Alternatively or additionally, the method 50 comprises controlling a user interface, which user interface is configured to enable user-inputting of an indication of a status of a respiratory disease being experienced by the subject, to issue a prompt to input the indication based on the output being provided.

Whilst not shown in Fig. 7, the method 50 may, for instance, further comprise storing the indication inputted via the user interface in a memory, as previously described.

Also provided is a computer program comprising computer program code which is adapted, when the computer program is run on a computer, to implement the above-described method 50. In a preferred embodiment, the computer program takes the form of an app, for example an app for a user device 40, such as a mobile device, e.g. tablet computer or a smart phone.

A clinical study was carried out in order to assess the factors influencing the probability of an asthma exacerbation. The following should be regarded as an explanatory and non-limiting example.

Albuterol administered using the ProAir Digihaler marketed by Teva Pharmaceutical Industries was utilized in this 12-week, open-label study, although the results of the study are more generally applicable to other rescue medicaments delivered using other device types.

Patients (≥18 years old) with exacerbation-prone asthma were recruited to the study. Patients used the ProAir Digihaler (albuterol 90 mcg as the sulfate with a lactose carrier, 1-2 inhalations every 4 hours) as needed.

The electronics module of the Digihaler recorded each use, i.e. each inhalation, and parameters relating to airflow during each inhalation: peak inspiratory flow, volume inhaled, time to peak flow and inhalation duration. Data were downloaded from the inhalers and, together with clinical data, subjected to a machine-learning algorithm to develop models predictive of an impending exacerbation.

The diagnosis of a clinical asthma exacerbation (CAE) in this example was based on the American Thoracic Society/European Respiratory Society statement (H.K. Reddel et al., Am J Respir Crit Care Med. 2009, 180(1), 59-99). It includes both a "severe CAE" or a "moderate CAE."

A severe CAE is defined as a CAE that involves worsening asthma that requires oral steroid (prednisone or equivalent) for at least three days and hospitalization. A moderate CAE requires oral steroid (prednisone or equivalent) for at least three days or hospitalization.

The objective and primary endpoint of the study was to explore the patterns and amount of albuterol use, as captured by the Digihaler, alone and in combination with other study data, such as the parameters relating to airflow during inhalation, physical activity, sleep, etc., preceding a CAE. This study represents the first successful attempt to develop a model to predict CAE derived from the use of a rescue medication inhaler device equipped with an integrated sensor and capable of measuring inhalation parameters.

Fig. 8 shows three timelines showing different inhalation patterns recorded for three different patients by their respective Digihalers. The uppermost timeline shows that the patient in question takes one inhalation at a time. The lowermost timeline shows that the patient in question takes two or more consecutive inhalations in a session. The term "session" is defined in this context as a sequence of inhalations with no more than 60 seconds between consecutive inhalations. The middle timeline shows that the patient in question inhales in various patterns. Thus, as well as recording the number of rescue inhalations, the Digihaler is configured to record the pattern of use.

It was found that 360 patients performed ≥1 valid inhalation from the Digihaler. These 360 patients were included in the analysis. Of these, 64 patients experienced a total of 78 CAEs.

A further clinical study was undertaken in order to better understand the factors influencing prediction of COPD exacerbation. The following should be regarded as an explanatory and non-limiting example.

Albuterol administered using the ProAir Digihaler marketed by Teva Pharmaceutical Industries was utilized in this 12-week, multicenter, open-label study, although the results of the study are more generally applicable to other rescue medicaments delivered using other device types.

The Digihaler enabled recording of: total number of inhalations, maximal inhalation flow, time to maximal inhalation flow, inhalation volume, and inhalation duration. The data were downloaded from the electronics module of the Digihaler at the end of the study.

An acute COPD exacerbation (AECOPD) was the primary outcome measure of this study. In this study, an AECOPD is an occurrence of either a "severe AECOPD" or a "moderate AECOPD." "Mild AECOPD" was not used as a measure of AECOPD in this study.

Severe AECOPD is defined as an event that involves worsening respiratory symptoms for at least two consecutive days requiring treatment with systemic corticosteroids (SCS, at least 10 mg prednisone equivalent above baseline) and/or systemic antibiotics, and a hospitalization for AECOPD.

Moderate AECOPD is defined as an event that involves worsening respiratory symptoms for at least two consecutive days requiring treatment with SCS (at least 10 mg prednisone equivalent above baseline), and/or systemic antibiotics, and an unscheduled encounter (such as a phone call, an office visit, an urgent care visit, or an emergency care visit) for a AECOPD, but not a hospitalization.

Patients (≥40 years old) with COPD were recruited to the study. Patients used the ProAir Digihaler (albuterol 90 mcg as the sulfate with a lactose carrier, 1-2 inhalations every 4 hours) as needed.

The inclusion criteria required that the patient is on a SABA plus at least one of the following: LABA, ICS/LABA, LAMA, or LABA/LAMA; suffered least one episode of moderate or severe AECOPD over the past 12 months before screening; is able to demonstrate appropriate use of albuterol from the Digihaler; and is willing to discontinue all other rescue or maintenance SABA or short-acting anti-muscarinic agents and replace them with the study-provided Digihaler for the duration of the trial.

Patients were excluded from the study if they had any clinically significant medical condition (treated or untreated) that, in the opinion of the investigator, would interfere with participation in the study; any other confounding underlying lung disorder other than COPD; used an investigational drug within 5 half-lives of it being discontinued, or 1 month of visit 2, whichever is longer; had congestive heart failure; were pregnant or were lactating, or had plans to become pregnant during the study.

Two subsets of ca. 100 patients were required to wear an accelerometer either on the ankle to measure physical activity (Total Daily Steps, TDS) or on the wrist to measure sleep disturbance (Sleep Disturbance Index, SDI).

The general factors of interest relating to rescue medicament use were:
(1) total number of inhalations in the days preceding the peak of a AECOPD
(2) number of days prior to the peak of a AECOPD when albuterol use increased, and
(3) number of albuterol uses in the 24 hours preceding a AECOPD.

Approximately 400 patients were enrolled. This provided 366 evaluable patients which completed the study. 336 valid inhalations of the Digihaler were recorded. Further details in this respect are provided in Table 1.

**Table 1**

| **Analysis group, n (%)** | **Total** |
|---|---|
| **Screened** | 423 |
| **Screen failure** | 18 |
| **Enrolled** | 405 (100) |
| **Enrolled but did not use ABS eMDPI** | 15 (4) |
| **Used ABS eMDPI at least once** | 390 (96) |
| **ITT analysis set** | 405 (100) |
| **Ankle accelerometry analysis set** | 96 (24) |
| **Wrist accelerometry analysis set** | 85 (21) |
| **Completed study** | 366 (90) |
| **Discontinued study** | 39 (10) |
| **Adverse event** | 8 (2) |
| **Death** | 2 (<1) |
| **Withdrawal by subject** | 14 (3) |
| **Non-compliance with study drug** | 1 (<1) |
| **Pregnancy** | 0 |
| **Lost to follow-up** | 3 (<1) |
| **Lack of efficacy** | 3 (<1) |
| **Protocol deviation** | 5 (1) |
| **Other** | 3 (<1) |

98 of the patients which completed the study suffered AECOPD events and used the Digihaler. A total of 121 moderate/severe AECOPD events were recorded. Further details are provided in Table 2.

**Table 2**

| | **AECOPD: "No"** | **AECOPD: "Yes, Moderate"** | **AECOPD: "Yes, Severe"** | **AECOPD: All** | **Overall** |
|---|---|---|---|---|---|
| **Number of Patients** | 287 | 85 | 24 | 109 | 396 |
| **Number of AECOPD events** | 0 | 95 | 26 | 121 | |
| **Number of patients with at least 1 AECOPD event** | 0 | 85 | 24 | 109 | |
| **Mean number of days Digihaler used by Patients** | 43.9 | 51.1 | 31.8 | 46.9 | 44.7 |
| **Min, max number of days Digihaler used by Patients** | 0, 92 | 0, 90 | 0, 85 | 0, 90 | 0, 92 |
| **Mean daily albuterol exposure (µg) of Patients** | 211.29 | 273.61 | 233.06 | 264.68 | 225.99 |
| **Min, max daily albuterol exposure (µg) of Patients** | 0.0, 1534.6 | 0.0, 1157.0 | 0.0, 1243.8 | 0.0, 1243.8 | 0.0, 1534.6 |

For 366 patients which completed the study: 30 (8%) patients did not use inhaler at all; 268 (73%) had a daily average of up to 5 inhalations; and 11 (3%) had a daily average greater than 10 inhalations.

Fig. 9 shows graphs of peak inhalation flow, inhalation volume, and number of rescue inhalations versus time for a subject suffering from COPD. The upper graph in Fig. 9 is the graph of peak inhalation flow versus time. This shows a decline in the peak inhalation flow over the first 40 to 50 days of the study. There is also a particularly pronounced decrease in inhalation volume (middle graph in Fig. 9) from around study day 40 to around study day 50, which is followed from around study day 50 onwards by increased frequency of rescue inhaler usage (lower graph in Fig. 9). The increased frequency of inhaler usage in this case coincides with continued decline in the peak inhalation flow and inhalation volume towards around study day 65 at which point a diagnosed COPD exacerbation 60 takes place.

It is evident from Fig. 9 that deterioration of the subject's lung condition, as indicated by the decrease in peak inhalation flow and inhalation volume during the first time period up to around study day 50, is followed by an increase in frequency of rescue inhaler usage during the second time period from around study day 50 to around study day 65. The frequency of rescue inhaler usage is greater during this second time period than during the first time period.

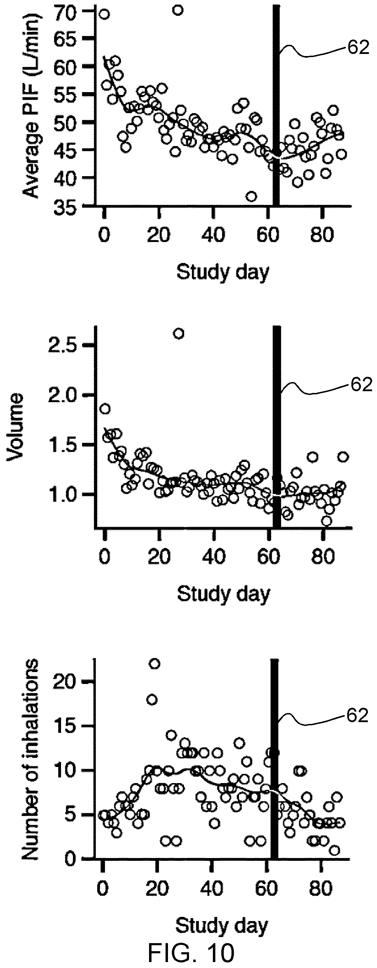

Fig. 10 shows graphs of inhalation volume, and number of rescue inhalations versus time for a subject suffering from asthma. The upper graph in Fig. 10 is the graph of peak inhalation flow versus time. This shows a marked decline in the peak inhalation flow, particularly over the first 5 days of the study. There is also a pronounced decrease in inhalation volume (middle graph in Fig. 10) from around study day 1 to around study day 5, which is followed from around study day 5 onwards by increased frequency of rescue inhaler usage (lower graph in Fig. 10). The increased frequency of inhaler usage in this case coincides with continued decline in the peak inhalation flow and inhalation volume towards around study day 65 at which point a diagnosed asthma exacerbation 62 takes place.

It is evident from Fig. 10 that deterioration of the subject's lung condition, as indicated by the decrease in peak inhalation flow and inhalation volume during the first time period up to around study day 5, is followed by an increase in frequency of rescue inhaler usage during the second time period from around study day 5 to around study day 65. The frequency of rescue inhaler usage is greater during this second time period than during the first time period.

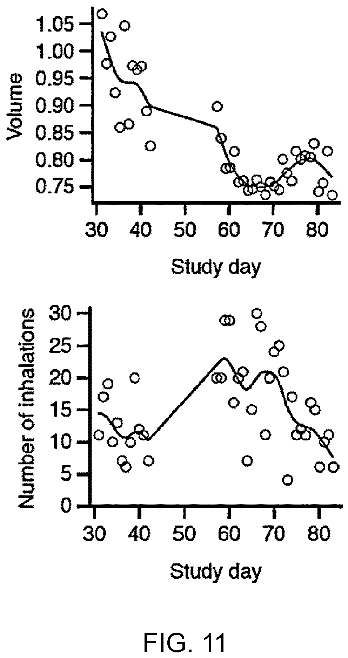

Fig. 11 shows graphs of inhalation volume, and number of rescue inhalations versus time for a subject suffering from asthma. The upper graph in Fig. 11 is the graph of inhalation volume versus time. This shows a marked decline in the inhalation volume between study day 30 and study day 40. This is followed from around study day 40 to study day 70 by increased frequency of rescue inhaler usage (lower graph in Fig. 11).

Whilst no formal exacerbation diagnosis was made for this subject, these data point to an asthma exacerbation having taken place around study day 70. Thus, Fig. 11 demonstrates asthma exacerbation identification based on the parameter, in this case inhalation volume, as a function of time being indicative of the lung condition of the subject deteriorating over the first time period (study day 30 to study day 40), and the frequency of the determined rescue inhalations being higher during the second time period (around study day 40 to study day 70) than during the first time period.

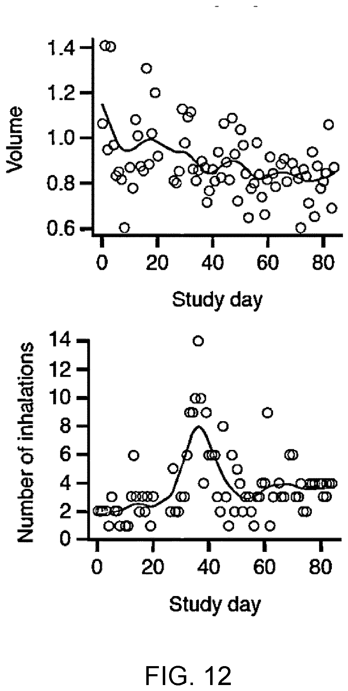

Fig. 12 shows graphs of inhalation volume, and number of rescue inhalations versus time for a subject suffering from COPD. The upper graph in Fig. 12 is the graph of inhalation volume versus time. This shows a marked decline in the inhalation volume between study day 1 and study day 20. This is followed from around study day 20 to around study day 40 by increased frequency of rescue inhaler usage (lower graph in Fig. 12).

Whilst, similarly to Fig. 11, no formal exacerbation diagnosis was made for this subject, these data point to a COPD exacerbation having taken place around study day 40. Thus, Fig. 12 demonstrates COPD exacerbation identification based on the parameter, in this case inhalation volume, as a function of time being indicative of the lung condition of the subject deteriorating over the first time period (study day 1 to study day 20), and the frequency of the determined rescue inhalations being higher during the second time period (around study day 20 to around study day 40) than during the first time period.

Figs. 13-16 provide a non-limiting example of an inhaler 100 which may be included in the system 10.

Fig. 13 provides a front perspective view of an inhaler 100 according to a non-limiting example. The inhaler 100 may, for example, be a breath-actuated inhaler. The inhaler 100 may include a top cap 102, a main housing 104, a mouthpiece 106, a mouthpiece cover 108, an electronics module 120, and an air vent 109. The mouthpiece cover 108 may be hinged to the main housing 104 so that it may open and close to expose the mouthpiece 106. Although illustrated as a hinged connection, the mouthpiece cover 108 may be connected to the inhaler 100 through other types of connections. Moreover, while the electronics module 120 is illustrated as housed within the top cap 102 at the top of the main housing 104, the electronics module 120 may be integrated and/or housed within the main body 104 of the inhaler 100.

The electronics module 120 may, for instance, include the above-described use determination system 12B and the transmission module 14. For example, the electronics module 120 may include a processor, memory configured to perform the functions of use determination system 12B and/or transmission module 14. The electronics module 120 may include switch(es), sensor(s), slider(s), and/or other instruments or measurement devices configured to determine inhaler usage information as described herein. The electronics module 120 may include a transceiver and/or other communication chips or circuits configured to perform the transmission functions of transmission module 14.

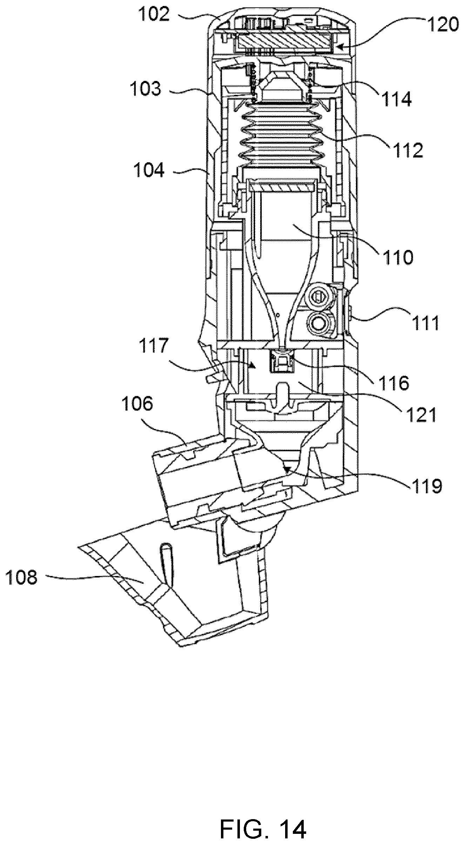

Fig. 14 provides a cross-sectional interior perspective view of the example inhaler 100. Inside the main housing 104, the inhalation device 100 may include a medication reservoir 110 and a dose delivery mechanism. For example, the inhaler 100 may include a medication reservoir 110 (e.g. a hopper), a bellows 112, a bellows spring 114, a yoke (not visible), a dosing cup 116, a dosing chamber 117, a deagglomerator 121, and a flow pathway 119. The medication reservoir 110 may include medication, such as dry powder medication, for delivery to the subject. Although illustrated as a combination of the bellows 112, the bellows spring 114, the yoke, the dosing cup 116, the dosing chamber 117, and the deagglomerator 121, the dose delivery mechanism may include a subset of the components described and/or the inhalation device 100 may include a different dose delivery mechanism (e.g. based on the type of inhalation device, the type of medication, etc.). For instance, in some examples the medication may be included in a blister strip and the dose delivery mechanism, which may include one or more wheels, levers, and/or actuators, is configured to advance the blister strip, open a new blister that includes a dose of medication, and make that dose of medication available to a dosing chamber and/or mouthpiece for inhalation by the user.

When the mouthpiece cover 108 is moved from the closed to the open position, the dose delivery mechanism of the inhaler 100 may prime a dose of medicament. In the illustrated example of Fig. 14, the mouthpiece cover 108 being moved from the closed to the open position may cause the bellows 112 to compress to deliver a dose of medication from the medication reservoir 110 to the dosing cup 116. Thereafter, a subject may inhale through the mouthpiece 106 in an effort to receive the dose of medication.

The airflow generated from the subject's inhalation may cause the deagglomerator 121 to aerosolize the dose of medication by breaking down the agglomerates of the medicament in the dose cup 116. The deagglomerator 121 may be configured to aerosolize the medication when the airflow through the flow pathway 119 meets or exceeds a particular rate, or is within a specific range. When aerosolized, the dose of medication may travel from the dosing cup 116, into the dosing chamber 117, through the flow pathway 119, and out of the mouthpiece 106 to the subject. If the airflow through the flow pathway 119 does not meet or exceed a particular rate, or is not within a specific range, the medication may remain in the dosing cup 116. In the event that the medication in the dosing cup 116 has not been aerosolized by the deagglomerator 121, another dose of medication may not be delivered from the medication reservoir 110 when the mouthpiece cover 108 is subsequently opened. Thus, a single dose of medication may remain in the dosing cup until the dose has been aerosolized by the deagglomerator 121. When a dose of medication is delivered, a dose confirmation may be stored in memory at the inhaler 100 as dose confirmation information.

As the subject inhales through the mouthpiece 106, air may enter the air vent to provide a flow of air for delivery of the medication to the subject. The flow pathway 119 may extend from the dosing chamber 117 to the end of the mouthpiece 106, and include the dosing chamber 117 and the internal portions of the mouthpiece 106. The dosing cup 116 may reside within or adjacent to the dosing chamber 117. Further, the inhaler 100 may include a dose counter 111 that is configured to be initially set to a number of total doses of medication within the medication reservoir 110 and to decrease by one each time the mouthpiece cover 108 is moved from the closed position to the open position.

The top cap 102 may be attached to the main housing 104. For example, the top cap 102 may be attached to the main housing 104 through the use of one or more clips that engage recesses on the main housing 104. The top cap 102 may overlap a portion of the main housing 104 when connected, for example, such that a substantially pneumatic seal exists between the top cap 102 and the main housing 104.

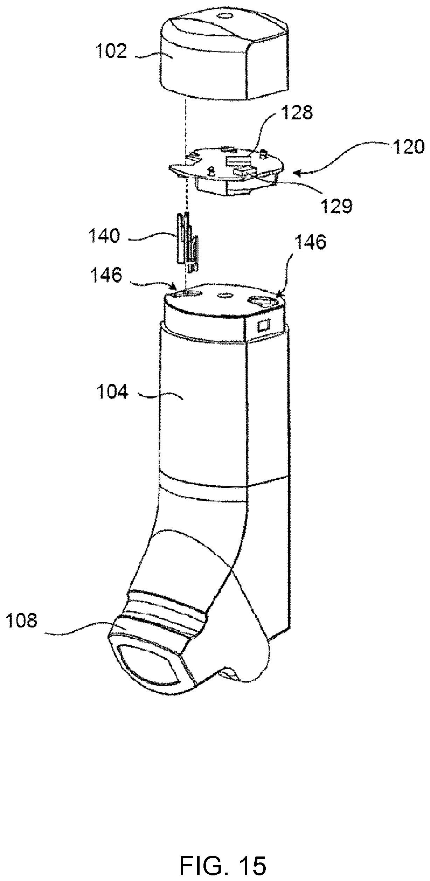

Fig. 15 is an exploded perspective view of the example inhaler 100 with the top cap 102 removed to expose the electronics module 120. As shown in Fig. 15, the top surface of the main housing 104 may include one or more (e.g. two) orifices 146. One of the orifices 146 may be configured to accept a slider 140. For example, when the top cap 102 is attached to the main housing 104, the slider 140 may protrude through the top surface of the main housing 104 via one of the orifices 146.

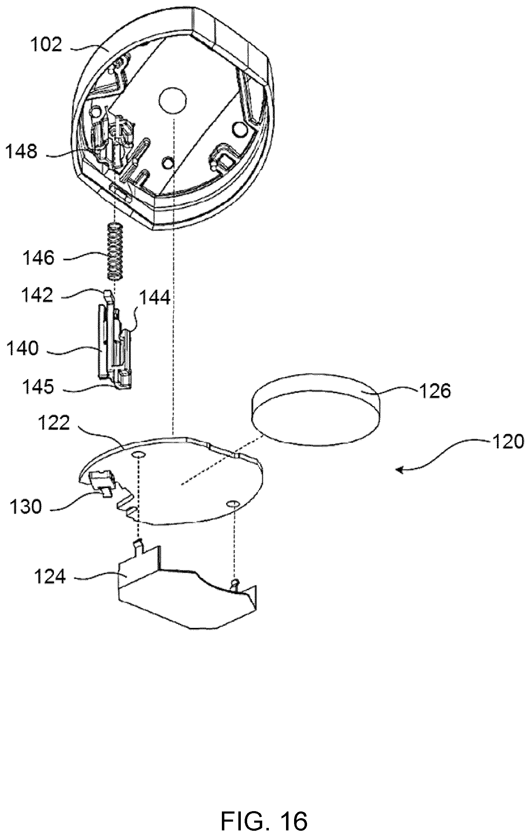

Fig. 16 is an exploded perspective view of the top cap 102 and the electronics module 120 of the example inhaler 100. As shown in Fig. 16, the slider 140 may define an arm 142, a stopper 144, and a distal end 145. The distal end 145 may be a bottom portion of the slider 140. The distal end 145 of the slider 140 may be configured to abut the yoke that resides within the main housing 104 (e.g. when the mouthpiece cover 108 is in the closed or partially open position). The distal end 145 may be configured to abut a top surface of the yoke when the yoke is in any radial orientation. For example, the top surface of the yoke may include a plurality of apertures (not shown), and the distal end 145 of the slider 140 may be configured to abut the top surface of the yoke, for example, whether or not one of the apertures is in alignment with the slider 140.

The top cap 102 may include a slider guide 148 that is configured to receive a slider spring 146 and the slider 140. The slider spring 146 may reside within the slider guide 148. The slider spring 146 may engage an inner surface of the top cap 102, and the slider spring 146 may engage (e.g. abut) an upper portion (e.g. a proximate end) of the slider 140. When the slider 140 is installed within the slider guide 148, the slider spring 146 may be partially compressed between the top of the slider 140 and the inner surface of the top cap 102. For example, the slider spring 146 may be configured such that the distal end 145 of the slider 140 remains in contact with the yoke when the mouthpiece cover 108 is closed. The distal end 145 of the slider 140 may also remain in contact with the yoke while the mouthpiece cover 108 is being opened or closed. The stopper 144 of the slider 140 may engage a stopper of the slider guide 148, for example, such that the slider 140 is retained within the slider guide 148 through the opening and closing of the mouthpiece cover 108, and vice versa. The stopper 144 and the slider guide 148 may be configured to limit the vertical (e.g. axial) travel of the slider 140. This limit may be less than the vertical travel of the yoke. Thus, as the mouthpiece cover 108 is moved to a fully open position, the yoke may continue to move in a vertical direction towards the mouthpiece 106 but the stopper 144 may stop the vertical travel of the slider 140 such that the distal end 145 of the slider 140 may no longer be in contact with the yoke.

More generally, the yoke may be mechanically connected to the mouthpiece cover 108 and configured to move to compress the bellows spring 114 as the mouthpiece cover 108 is opened from the closed position and then release the compressed bellows spring 114 when the mouthpiece cover reaches the fully open position, thereby causing the bellows 112 to deliver the dose from the medication reservoir 110 to the dosing cup 116. The yoke may be in contact with the slider 140 when the mouthpiece cover 108 is in the closed position. The slider 140 may be arranged to be moved by the yoke as the mouthpiece cover 108 is opened from the closed position and separated from the yoke when the mouthpiece cover 108 reaches the fully open position. This arrangement may be regarded as a non-limiting example of the previously described dose metering assembly, since opening the mouthpiece cover 108 causes the metering of the dose of the medicament.

The movement of the slider 140 during the dose metering may cause the slider 140 to engage and actuate a switch 130. The switch 130 may trigger the electronics module 120 to register the dose metering. The slider 140 and switch 130 together with the electronics module 120 may thus be regarded as being included in the use determination system 12B described above. The slider 140 may be regarded in this example as the means by which the use determination system 12B is configured to register the metering of the dose by the dose metering assembly, each metering being thereby indicative of the inhalation performed by the subject using the inhaler 100.

Actuation of the switch 130 by the slider 140 may also, for example, cause the electronics module 120 to transition from the first power state to a second power state, and to sense an inhalation by the subject from the mouthpiece 106.

The electronics module 120 may include a printed circuit board (PCB) assembly 122, a switch 130, a power supply (e.g. a battery 126), and/or a battery holder 124. The PCB assembly 122 may include surface mounted components, such as a sensor system 128, a wireless communication circuit 129, the switch 130, and or one or more indicators (not shown), such as one or more light emitting diodes (LEDs). The electronics module 120 may include a controller (e.g. a processor) and/or memory. The controller and/or memory may be physically distinct components of the PCB 122. Alternatively, the controller and memory may be part of another chipset mounted on the PCB 122, for example, the wireless communication circuit 129 may include the controller and/or memory for the electronics module 120. The controller of the electronics module 120 may include a microcontroller, a programmable logic device (PLD), a microprocessor, an application specific integrated circuit (ASIC), a field programmable gate array (FPGA), or any suitable processing device or control circuit.

The controller may access information from, and store data in the memory. The memory may include any type of suitable memory, such as non-removable memory and/or removable memory. The non-removable memory may include random-access memory (RAM), read-only memory (ROM), a hard disk, or any other type of memory storage device. The removable memory may include a subscriber identity module (SIM) card, a memory stick, a secure digital (SD) memory card, and the like. The memory may be internal to the controller. The controller may also access data from, and store data in, memory that is not physically located within the electronics module 120, such as on a server or a smart phone.

The sensor system 128 may include one or more sensors. The sensor system 128 may be, for example, included in the sensor system 12A and use determination system 12B described above. The sensor system 128 may include one or more sensors, for example, of different types, such as, but not limited to one or more pressure sensors, temperature sensors, humidity sensors, orientation sensors, acoustic sensors, and/or optical sensors. The one or more pressure sensors may include a barometric pressure sensor (e.g. an atmospheric pressure sensor), a differential pressure sensor, an absolute pressure sensor, and/or the like. The sensors may employ microelectromechanical systems (MEMS) and/or nanoelectromechanical systems (NEMS) technology. The sensor system 128 may be configured to provide an instantaneous reading (e.g. pressure reading) to the controller of the electronics module 120 and/or aggregated readings (e.g. pressure readings) over time. As illustrated in Figs. 14 and 15, the sensor system 128 may reside outside the flow pathway 119 of the inhaler 100, but may be pneumatically coupled to the flow pathway 119.

The controller of the electronics module 120 may receive signals corresponding to measurements from the sensor system 128. The controller may calculate or determine one or more airflow metrics using the signals received from the sensor system 128. The airflow metrics may be indicative of a profile of airflow through the flow pathway 119 of the inhaler 100. For example, if the sensor system 128 records a change in pressure of 0.3 kilopascals (kPa), the electronics module 120 may determine that the change corresponds to an airflow rate of approximately 45 liters per minute (Lpm) through the flow pathway 119.

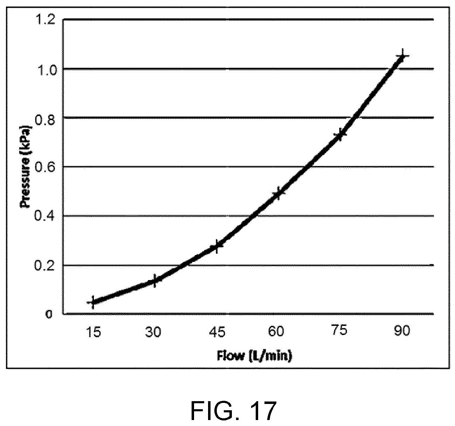

Fig. 17 shows a graph of airflow rates versus pressure. The airflow rates and profile shown in Fig. 17 are merely examples and the determined rates may depend on the size, shape, and design of the inhalation device 100 and its components.

The processing module 14 may generate personalized data in real-time by comparing signals received from the sensor system 128 and/or the determined airflow metrics to one or more thresholds or ranges, for example, as part of an assessment of how the inhaler 100 is being used and/or whether the use is likely to result in the delivery of a full dose of medication. For example, where the determined airflow metric corresponds to an inhalation with an airflow rate below a particular threshold, the processing module 14 may determine that there has been no inhalation or an insufficient inhalation from the mouthpiece 106 of the inhaler 100. If the determined airflow metric corresponds to an inhalation with an airflow rate above a particular threshold, the processing module 14 may determine that there has been an excessive inhalation from the mouthpiece 106. If the determined airflow metric corresponds to an inhalation with an airflow rate within a particular range, the processing module 14 may determine that the inhalation is "good", or likely to result in a full dose of medication being delivered.

The pressure measurement readings and/or the computed airflow metrics may be indicative of the quality or strength of inhalation from the inhaler 100. For example, when compared to a particular threshold or range of values, the readings and/or metrics may be used to categorize the inhalation as a certain type of event, such as a good inhalation event, a low inhalation event, a no inhalation event, or an excessive inhalation event. The categorization of the inhalation may be usage parameters stored as personalized data of the subject.

The no or low inhalation event may be associated with pressure measurement readings and/or airflow metrics below a particular threshold, such as an airflow rate less than or equal to 30 Lpm. The no inhalation event may occur when a subject does not inhale from the mouthpiece 106 after opening the mouthpiece cover 108 and during the measurement cycle. The no or low inhalation event may also occur when the subject's inspiratory effort is insufficient to ensure proper delivery of the medication via the flow pathway 119, such as when the inspiratory effort generates insufficient airflow to activate the deagglomerator 121 and, thus, aerosolize the medication in the dosing cup 116.

A fair inhalation event may be associated with pressure measurement readings and/or airflow metrics within a particular range, such as an airflow rate greater than 30 Lpm and less than or equal to 45 Lpm. The fair inhalation event may occur when the subject inhales from the mouthpiece 106 after opening the mouthpiece cover 108 and the subject's inspiratory effort causes at least a partial dose of the medication to be delivered via the flow pathway 119. That is, the inhalation may be sufficient to activate the deagglomerator 121 such that at least a portion of the medication is aerosolized from the dosing cup 116.

The good inhalation event may be associated with pressure measurement readings and/or airflow metrics above the low inhalation event, such as an airflow rate which is greater than 45 Lpm and less than or equal to 200 Lpm. The good inhalation event may occur when the subject inhales from the mouthpiece 106 after opening the mouthpiece cover 108 and the subject's inspiratory effort is sufficient to ensure proper delivery of the medication via the flow pathway 119, such as when the inspiratory effort generates sufficient airflow to activate the deagglomerator 121 and aerosolize a full dose of medication in the dosing cup 116.

The excessive inhalation event may be associated with pressure measurement readings and/or airflow metrics above the good inhalation event, such as an airflow rate above 200 Lpm. The excessive inhalation event may occur when the subject's inspiratory effort exceeds the normal operational parameters of the inhaler 100. The excessive inhalation event may also occur if the device 100 is not properly positioned or held during use, even if the subject's inspiratory effort is within a normal range. For example, the computed airflow rate may exceed 200 Lpm if the air vent is blocked or obstructed (e.g. by a finger or thumb) while the subject is inhaling from the mouthpiece 106.

Any suitable thresholds or ranges may be used to categorize a particular event. Some or all of the events may be used. For example, the no inhalation event may be associated with an airflow rate which is less than or equal to 45 Lpm and the good inhalation event may be associated with an airflow rate which is greater than 45 Lpm and less than or equal to 200 Lpm. As such, the low or fair inhalation event may not be used at all in some cases.

The pressure measurement readings and/or the computed airflow metrics may also be indicative of the direction of flow through the flow pathway 119 of the inhaler 100. For example, if the pressure measurement readings reflect a negative change in pressure, the readings may be indicative of air flowing out of the mouthpiece 106 via the flow pathway 119. If the pressure measurement readings reflect a positive change in pressure, the readings may be indicative of air flowing into the mouthpiece 106 via the flow pathway 119. Accordingly, the pressure measurement readings and/or airflow metrics may be used to determine whether a subject is exhaling into the mouthpiece 106, which may signal that the subject is not using the device 100 properly.

The inhaler 100 may include a spirometer or similarly operating device to enable measurement of lung function metrics. For example, the inhaler 100 may perform measurements to obtain metrics related to a subject's lung capacity. The spirometer or similarly operating device may measure the volume of air inhaled and/or exhaled by the subject. The spirometer or similarly operating device may use pressure transducers, ultrasound, or a water gauge to detect the changes in the volume of air inhaled and/or exhaled.

The personalized data collected from, or calculated based on, the usage of the inhaler 100 (e.g. pressure metrics, airflow metrics, lung function metrics, dose confirmation information, etc.) may be computed and/or assessed via external devices as well (e.g. partially or entirely). More specifically, the wireless communication circuit 129 in the electronics module 120 may include a transmitter and/or receiver (e.g. a transceiver), as well as additional circuity.

For example, the wireless communication circuit 129 may include a Bluetooth chip set (e.g. a Bluetooth Low Energy chip set), a ZigBee chipset, a Thread chipset, etc. As such, the electronics module 120 may wirelessly provide the personalized data, such as pressure measurements, airflow metrics, lung function metrics, dose confirmation information, and/or other conditions related to usage of the inhaler 100, to an external processing module 14, such as a processing module 14 included in a smart phone 40. The personalized data may be provided in real time to the external device to enable acute risk level determination based on real-time data from the inhaler 100 that indicates time of use, how the inhaler 100 is being used, and personalized data about the subject, such as real-time data related to the subject's lung function and/or medical treatment. The external device may include software for processing the received information and for providing compliance and adherence feedback to the subject via a graphical user interface (GUI). The graphical user interface may be included in, or may define, the user interface 13 included in the system 10.

The airflow metrics may include personalized data that is collected from the inhaler 100 in real-time, such as one or more of an average flow of an inhalation/exhalation, a peak flow of an inhalation/exhalation (e.g. a maximum inhalation received), a volume of an inhalation/exhalation, a time to peak of an inhalation/exhalation, and/or the duration of an inhalation/exhalation. The airflow metrics may also be indicative of the direction of flow through the flow pathway 119. That is, a negative change in pressure may correspond to an inhalation from the mouthpiece 106, while a positive change in pressure may correspond to an exhalation into the mouthpiece 106. When calculating the airflow metrics, the electronics module 120 may be configured to eliminate or minimize any distortions caused by environmental conditions. For example, the electronics module 120 may re-zero to account for changes in atmospheric pressure before or after calculating the airflow metrics. The one or more pressure measurements and/or airflow metrics may be time-stamped and stored in the memory of the electronics module 120.

In addition to the airflow metrics, the inhaler 100, or another computing device, may use the airflow metrics to generate additional personalized data. For example, the controller of the electronics module 120 of the inhaler 100 and/or the processing module 14 may translate the airflow metrics into other metrics that indicate the subject's lung function and/or lung health that are understood to medical practitioners, such as peak inspiratory flow metrics, peak expiratory flow metrics, and/or forced expiratory volume in 1 second (FEV1), for example. The processing module 14 and/or the electronics module 120 of the inhaler 100 may determine a measure of the subject's lung function and/or lung health using a mathematical model such as a regression model. The mathematical model may identify a correlation between the total volume of an inhalation and FEV1. The mathematical model may identify a correlation between peak inspiratory flow and FEV1. The mathematical model may identify a correlation between the total volume of an inhalation and peak expiratory flow. The mathematical model may identify a correlation between peak inspiratory flow and peak expiratory flow.

The battery 126 may provide power to the components of the PCB 122. The battery 126 may be any suitable source for powering the electronics module 120, such as a coin cell battery, for example. The battery 126 may be rechargeable or non-rechargeable. The battery 126 may be housed by the battery holder 124. The battery holder 124 may be secured to the PCB 122 such that the battery 126 maintains continuous contact with the PCB 122 and/or is in electrical connection with the components of the PCB 122. The battery 126 may have a particular battery capacity that may affect the life of the battery 126. As will be further discussed below, the distribution of power from the battery 126 to the one or more components of the PCB 122 may be managed to ensure the battery 126 can power the electronics module 120 over the useful life of the inhaler 100 and/or the medication contained therein.

In a connected state, the communication circuit and memory may be powered on and the electronics module 120 may be "paired" with an external device, such as a smart phone. The controller may retrieve data from the memory and wirelessly transmit the data to the external device. The controller may retrieve and transmit the data currently stored in the memory. The controller may also retrieve and transmit a portion of the data currently stored in the memory. For example, the controller may be able to determine which portions have already been transmitted to the external device and then transmit the portion(s) that have not been previously transmitted. Alternatively, the external device may request specific data from the controller, such as any data that has been collected by the electronics module 120 after a particular time or after the last transmission to the external device. The controller may retrieve the specific data, if any, from the memory and transmit the specific data to the external device.

The data stored in the memory of the electronics module 120 (e.g. the signals generated by the switch 130, the pressure measurement readings taken by the sensory system 128 and/or the airflow metrics computed by the controller of the PCB 122) may be transmitted to an external device, which may process and analyze the data to determine the usage parameters associated with the inhaler 100. Further, a mobile application residing on the mobile device may generate feedback for the user based on data received from the electronics module 120. For example, the mobile application may generate daily, weekly, or monthly report, provide confirmation of error events or notifications, provide instructive feedback to the subject, and/or the like.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Any reference signs in the claims should not be construed as limiting the scope.

## Claims

1. A system for providing an output for warning of a respiratory disease exacerbation in a subject, the system comprising:
a first inhaler for delivering a rescue medicament to the subject, the first inhaler having a use determination system configured to determine a rescue inhalation performed by the subject using the first inhaler;
an optional second inhaler for delivering a maintenance medicament to the subject during a routine inhalation,
wherein the system comprises a sensor system configured to measure a parameter relating to airflow during said rescue inhalation and/or during said routine inhalation using the second inhaler when included in the system; and
a processing module configured to:
monitor a frequency of the determined rescue inhalations;
monitor the parameter as a function of time, and
provide the output if the parameter as a function of time is indicative of the lung condition of the subject deteriorating over a first time period, and the frequency of the determined rescue inhalations is higher during a second time period than during the first time period, the second time period being subsequent to the first time period.

2. The system according to claim 1, further comprising a user interface, wherein the processing module is configured to control the user interface to issue an exacerbation warning based on said output being provided.

3. The system according to claim 1, further comprising a user interface configured to enable user-inputting of an indication of a status of a respiratory disease being experienced by the subject, and wherein the processing module is configured to control the user interface to issue a prompt to input said indication based on said output being provided.

4. The system according to claim 3, wherein the processing module is configured to control the user interface to issue an exacerbation warning based on said output being provided.

5. The system according to claim 3 or claim 4, wherein the system comprises a memory for storing said indication inputted via the user interface.

6. The system according to any of claims 3 to 5, wherein the user interface is configured to provide a plurality of user-selectable respiratory disease status options, wherein the indication is defined by user-selection of at least one of said status options.

7. The system according to claim 6, wherein the user interface is configured to provide said status options in the form of selectable icons, checkboxes, a slider, and/or a dial.

8. The system according to any of claims 1 to 7, wherein the parameter is at least one of a peak inhalation flow, an inhalation volume, a time to peak inhalation flow, and an inhalation duration.

9. The system according to any of claims 1 to 8, wherein the sensor system comprises a pressure sensor; optionally wherein the use determination system comprises a further pressure sensor, the pressure sensor and the further pressure sensor being the same as or different from each other.

10. The system according to any of claims 1 to 9, wherein the use determination system comprises a mechanical switch configured to be actuated prior to, during, or after use of the first inhaler.

11. The system according to any of claims 1 to 10, wherein the first inhaler comprises:
a medicament reservoir; and
a dose metering assembly configured to meter a dose of said medicament from the reservoir, wherein the use determination system is configured to register the metering of said dose by the dose metering assembly, each metering being thereby indicative of said rescue inhalation performed by the subject using the first inhaler.

12. The system according to any of claims 1 to 11, wherein the first inhaler is configured to deliver a rescue medicament selected from albuterol, formoterol, budesonide combined with formoterol, beclomethasone combined with albuterol, and fluticasone combined with albuterol; optionally wherein the second inhaler, when present, is configured to deliver a maintenance medicament selected from budesonide, beclomethasone, fluticasone, and salmeterol combined with fluticasone.

13. A computer-implemented method for providing an output for warning of a respiratory disease exacerbation in a subject, the method comprising:
monitoring a frequency of rescue inhalations using a first inhaler;
monitoring a parameter relating to airflow as a function of time, the parameter being sensed during the rescue inhalations and/or during routine inhalations of a maintenance medicament performed by the subject using a second inhaler; and
providing the output if the parameter as a function of time is indicative of the lung condition of the subject deteriorating over a first time period, and the frequency of rescue inhalations is higher during a second time period than during the first time period, the second time period being subsequent to the first time period.

14. The method according to claim 13, further comprising controlling a user interface to issue an exacerbation warning based on said output being provided.

15. A computer program comprising computer program code which is adapted, when said program is run on a computer, to implement the method of claim 13 or 14.

## Patentansprüche

1. System zum Bereitstellen einer Ausgabe zum Warnen vor einer Exazerbation einer Atemwegserkrankung bei einem Subjekt, wobei das System Folgendes umfasst:
einen ersten Inhalator zum Abgeben eines Notfallmedikaments an das Subjekt, wobei der erste Inhalator ein Verwendungsbestimmungssystem aufweist, das dazu konfiguriert ist, eine durch das Subjekt unter Verwendung des ersten Inhalators durchgeführte Notfallinhalation zu bestimmen;
einen optionalen zweiten Inhalator zum Abgeben eines Erhaltungsmedikaments an das Subjekt während einer Routineinhalation,
wobei das System ein Sensorsystem umfasst, das dazu konfiguriert ist, einen Parameter in Bezug auf einen Luftstrom während der Notfallinhalation und/oder während der Routineinhalation unter Verwendung des zweiten Inhalators zu messen, wenn er in dem System enthalten ist; und
ein Verarbeitungsmodul, das zu Folgendem konfiguriert ist:
Überwachen einer Häufigkeit der bestimmten Notfallinhalationen;
Überwachen des Parameters in Abhängigkeit von der Zeit und
Bereitstellen der Ausgabe, falls der Parameter in Abhängigkeit von der Zeit angibt, dass sich der Lungenzustand des Subjekts über einen ersten Zeitraum verschlechtert, und die Häufigkeit der bestimmten Notfallinhalationen während eines zweiten Zeitraums größer als während des ersten Zeitraums ist, wobei der zweite Zeitraum auf den ersten Zeitraum folgt.

2. System nach Anspruch 1, ferner umfassend eine Benutzerschnittstelle, wobei das Verarbeitungsmodul dazu konfiguriert ist, die Benutzerschnittstelle so zu steuern, dass sie eine Exazerbationswarnung basierend darauf, dass die Ausgabe bereitgestellt wird, herausgibt.

3. System nach Anspruch 1, ferner umfassend eine Benutzerschnittstelle, die dazu konfiguriert ist, eine Benutzereingabe einer Angabe eines Status einer Atemwegserkrankung, an der das Subjekt leidet, zu ermöglichen, und wobei das Verarbeitungsmodul dazu konfiguriert ist, die Benutzerschnittstelle so zu steuern, dass sie eine Aufforderung zum Eingeben der Angabe basierend darauf, dass die Ausgabe bereitgestellt wird, herausgibt.

4. System nach Anspruch 3, wobei das Verarbeitungsmodul dazu konfiguriert ist, die Benutzerschnittstelle so zu steuern, dass sie eine Exazerbationswarnung basierend darauf, dass die Ausgabe bereitgestellt wird, herausgibt.

5. System nach Anspruch 3 oder Anspruch 4, wobei das System einen Speicher zum Speichern der über die Benutzerschnittstelle eingegebenen Angabe umfasst.

6. System nach einem der Ansprüche 3 bis 5, wobei die Benutzerschnittstelle dazu konfiguriert ist, eine Vielzahl von durch den Benutzer auswählbaren Statusoptionen von Atemwegserkrankungen bereitzustellen, wobei die Angabe durch Benutzerauswahl mindestens einer der Statusoptionen definiert ist.

7. System nach Anspruch 6, wobei die Benutzerschnittstelle dazu konfiguriert ist, die Statusoptionen in Form von auswählbaren Symbolen, Markierungsfeldern, einem Schieberegler und/oder einer Skalenscheibe bereitzustellen.

8. System nach einem der Ansprüche 1 bis 7, wobei der Parameter mindestens einer von einem Peak-Inhalationsfluss, einem Inhalationsvolumen, einer Zeit bis zum Peak-Inhalationsfluss und einer Inhalationsdauer ist.

9. System nach einem der Ansprüche 1 bis 8, wobei das Sensorsystem einen Drucksensor umfasst; wobei optional das Verwendungsbestimmungssystem einen weiteren Drucksensor umfasst, wobei der Drucksensor und der weitere Drucksensor gleich oder voneinander verschieden sind.

10. System nach einem der Ansprüche 1 bis 9, wobei das Verwendungsbestimmungssystem einen mechanischen Schalter umfasst, der dazu konfiguriert ist, vor, während oder nach Verwendung des ersten Inhalators betätigt zu werden.

11. System nach einem der Ansprüche 1 bis 10, wobei der erste Inhalator Folgendes umfasst:
einen Medikamentenbehälter; und
eine Dosiszumessungsbaugruppe, die dazu konfiguriert ist, eine Dosis des Medikaments aus dem Behälter zuzumessen, wobei das Verwendungsbestimmungssystem dazu konfiguriert ist, das Zumessen der Dosis durch die Dosiszumessungsbaugruppe zu registrieren, wobei jedes Zumessen dadurch die Notfallinhalation angibt, die durch das Subjekt unter Verwendung des ersten Inhalators durchgeführt wird.

12. System nach einem der Ansprüche 1 bis 11, wobei der erste Inhalator dazu konfiguriert ist, ein Notfallmedikament abzugeben, das aus Albuterol, Formoterol, Budesonid in Kombination mit Formoterol, Beclomethason in Kombination mit Albuterol und Fluticason in Kombination mit Albuterol ausgewählt ist; optional wobei der zweite Inhalator, wenn vorhanden, dazu konfiguriert ist, ein Erhaltungsmedikament abzugeben, das aus Budesonid, Beclomethason, Fluticason und Salmeterol in Kombination mit Fluticason ausgewählt ist.

13. Computerimplementiertes Verfahren zum Bereitstellen einer Ausgabe zum Warnen vor einer Exazerbation einer Atemwegserkrankung bei einem Subjekt, wobei das Verfahren Folgendes umfasst:
Überwachen einer Häufigkeit von Notfallinhalationen unter Verwendung eines ersten Inhalators;
Überwachen eines Parameters in Bezug auf einen Luftstrom in Abhängigkeit von der Zeit, wobei der Parameter während der Notfallinhalationen und/oder während Routineinhalationen eines Erhaltungsmedikaments erfasst wird, die durch das Subjekt unter Verwendung eines zweiten Inhalators durchgeführt werden; und
Bereitstellen der Ausgabe, falls der Parameter in Abhängigkeit von der Zeit angibt, dass sich der Lungenzustand des Subjekts über einen ersten Zeitraum verschlechtert, und die Häufigkeit der Notfallinhalationen während eines zweiten Zeitraums größer als während des ersten Zeitraums ist, wobei der zweite Zeitraum auf den ersten Zeitraum folgt.

14. Verfahren nach Anspruch 13, ferner umfassend Steuern einer Benutzerschnittstelle, sodass sie eine Exazerbationswarnung basierend darauf, dass die Ausgabe bereitgestellt wird, herausgibt.

15. Computerprogramm, einen Computerprogrammcode umfassend, der dazu ausgelegt ist, wenn das Programm auf einem Computer läuft, das Verfahren nach Anspruch 13 oder 14 zu implementieren.

## Revendications

1. Système permettant de fournir une sortie destinée à avertir d'une exacerbation de maladie respiratoire chez un sujet, le système comprenant :
un premier inhalateur permettant d'administrer un médicament de secours au sujet, le premier inhalateur comportant un système de détermination d'utilisation conçu pour déterminer une inhalation de secours effectuée par le sujet à l'aide du premier inhalateur ;
un second inhalateur éventuel permettant d'administrer un médicament d'entretien au sujet lors d'une inhalation de routine,
ledit système comprenant un système de capteur conçu pour mesurer un paramètre relatif au flux d'air pendant ladite inhalation de secours et/ou pendant ladite inhalation de routine à l'aide du second inhalateur lorsqu'il est inclus dans le système ; et
un module de traitement conçu pour :
surveiller une fréquence des inhalations de secours déterminées ;
surveiller le paramètre en fonction du temps, et
fournir la sortie si le paramètre en fonction du temps est révélateur de l'état pulmonaire du sujet se détériorant sur une première période de temps, et la fréquence des inhalations de secours déterminées est plus élevée pendant une seconde période de temps que pendant la première période de temps, la seconde période de temps étant consécutive à la première période de temps.

2. Système selon la revendication 1, comprenant en outre une interface utilisateur, ledit module de traitement étant conçu pour commander à l'interface utilisateur d'émettre un avertissement d'exacerbation sur la base de ladite sortie fournie.

3. Système selon la revendication 1, comprenant en outre une interface utilisateur conçue pour permettre à l'utilisateur d'entrer une indication d'un état d'une maladie respiratoire dont souffre le sujet, et ledit module de traitement étant conçu pour commander à l'interface utilisateur d'émettre une invite à entrer ladite indication sur la base de ladite sortie fournie.

4. Système selon la revendication 3, ledit module de traitement étant conçu pour commander à l'interface utilisateur d'émettre un avertissement d'exacerbation sur la base de ladite sortie fournie.

5. Système selon la revendication 3 ou la revendication 4, ledit système comprenant une mémoire destinée à stocker ladite indication entrée par l'intermédiaire de l'interface utilisateur.

6. Système selon l'une quelconque des revendications 3 à 5, ladite interface utilisateur étant conçue pour fournir une pluralité d'options d'état de maladie respiratoire sélectionnables par l'utilisateur, ladite indication étant définie par la sélection par l'utilisateur d'au moins l'une desdites options d'état.

7. Système selon la revendication 6, ladite interface utilisateur étant conçue pour fournir lesdites options d'état sous la forme d'icônes sélectionnables, de cases à cocher, d'un curseur et/ou d'un cadran.

8. Système selon l'une quelconque des revendications 1 à 7, ledit paramètre étant au moins un parmi un débit d'inhalation maximal, un volume d'inhalation, un temps jusqu'au débit d'inhalation maximal et une durée d'inhalation.

9. Système selon l'une quelconque des revendications 1 à 8, ledit système de capteur comprenant un capteur de pression ; éventuellement ledit système de détermination d'utilisation comprenant un autre capteur de pression, le capteur de pression et l'autre capteur de pression étant identiques l'un à l'autre ou différents l'un de l'autre.

10. Système selon l'une quelconque des revendications 1 à 9, ledit système de détermination d'utilisation comprenant un interrupteur mécanique conçu pour être actionné avant, pendant ou après l'utilisation du premier inhalateur.

11. Système selon l'une quelconque des revendications 1 à 10, ledit premier inhalateur comprenant :
un réservoir de médicament ; et
un ensemble de mesure de dose conçu pour mesurer une dose dudit médicament à partir du réservoir, ledit système de détermination d'utilisation étant conçu pour enregistrer la mesure de ladite dose par l'ensemble de mesure de dose, chaque mesure indiquant ainsi ladite inhalation de secours effectuée par le sujet à l'aide du premier inhalateur.

12. Système selon l'une quelconque des revendications 1 à 11, ledit premier inhalateur étant conçu pour administrer un médicament de secours choisi parmi l'albutérol, le formotérol, le budésonide combiné au formotérol, la béclométhasone combinée à l'albutérol et la fluticasone combinée à l'albutérol ; éventuellement ledit second inhalateur, lorsqu'il est présent, étant conçu pour administrer un médicament d'entretien choisi parmi le budésonide, la béclométhasone, la fluticasone et le salmétérol combiné à la fluticasone.

13. Procédé mis en œuvre par ordinateur permettant de fournir une sortie destinée à avertir d'une exacerbation de maladie respiratoire chez un sujet, le procédé comprenant :
la surveillance d'une fréquence des inhalations de secours à l'aide d'un premier inhalateur ;
la surveillance d'un paramètre relatif au flux d'air en fonction du temps, le paramètre étant détecté pendant les inhalations de secours et/ou pendant les inhalations de routine d'un médicament d'entretien effectuées par le sujet à l'aide d'un second inhalateur ; et
la fourniture de la sortie si le paramètre en fonction du temps est révélateur de l'état pulmonaire du sujet se détériorant sur une première période de temps, et la fréquence des inhalations de secours est plus élevée pendant une seconde période de temps que pendant la première période de temps, la seconde période de temps étant consécutive à la première période de temps.

14. Procédé selon la revendication 13, comprenant en outre la commande à une interface utilisateur d'émettre un avertissement d'exacerbation sur la base de ladite sortie fournie.

15. Programme informatique comprenant un code de programme informatique qui est adapté, lorsque ledit programme est exécuté sur un ordinateur, à la mise en œuvre du procédé selon la revendication 13 ou 14.
